# EUROPÄISCHE PATENTANMELDUNG

(11) **EP 1 632 493 A1**
(43) Veröffentlichungstag der Anmeldung: **08.03.2006**
(21) Anmeldenummer: 04020125.3
(22) Anmeldetag: 25.08.2004
(51) Int. Cl.: C07D 475/04, A61K 31/4375, A61P 35/00

(54) **Dihydropteridinonderivative, Verfahren zu deren Herstellung und deren Verwendung als Arzneimittel**

(71) Anmelder: Boehringer Ingelheim Pharma GmbH & Co.KG, 55216 Ingelheim am Rhein (DE)
(72) Erfinder: Die Erfindernennung liegt noch nicht vor

(57) **Zusammenfassung**

Die vorliegende Erfindung betrifft neue Dihydropteridinone der allgemeinen Formel (I) 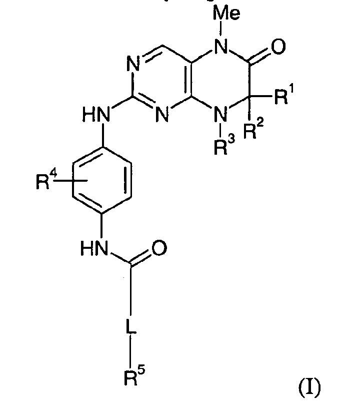 wobei die Reste L, R¹, R², R³, R⁴ und R⁵ die in den Ansprüchen und der Beschreibung genannten Bedeutungen haben, deren Isomere, Verfahren zur Herstellung dieser Dihydropteridinone sowie deren Verwendung als Arzneimittel.

## Beschreibung

Die vorliegende Erfindung betrifft neue Dihydropteridinone der allgemeinen Formel (I) 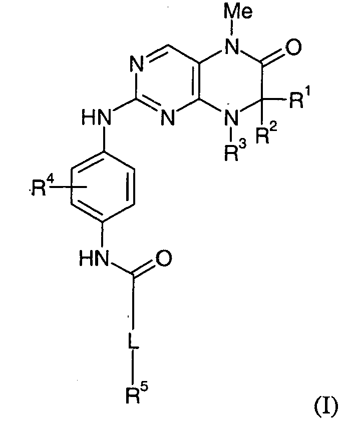
wobei die Reste L, R¹, R², R³, R⁴ und R⁵ die in den Ansprüchen und der Beschreibung genannten Bedeutungen haben, deren Isomere, Verfahren zur Herstellung dieser Dihydropteridinone sowie deren Verwendung als Arzneimittel.

### Hintergrund der Erfindung

Pteridinon-Derivate sind als Wirkstoffe mit antiproliferativer Wirkung aus dem Stand der Technik bekannt. WO 01/019825 und WO 03/020722 beschreiben die Verwendung von Pteridinonderivaten zur Behandlung von Tumorerkrankungen.
Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).
Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu et al. 2000, *Cell* **102**:279-91). Ein spezifischer Zellzyklusarrest in der G2/M Phase kann aber auch z.B. durch Inhibition von spezifischen Phosphatasen wie z.B. Cdc25C (Russell and Nurse 1986, *Cell* **45**:145-53) ausgelöst werden. Hefen mit defektem Cdc25 Gen arretieren in der G2 Phase, während eine Überexpression von Cdc25 zu einem verfrühten Eintritt in die Mitosephase führt (Russell und Nurse 1987, *Cell* **49**:559-67). Desweiteren kann ein Arrest in der G2/M Phase auch durch Inhibition von bestimmten Motorproteinen, den so genannten Kinesinen wie z.B. Eg5 (Mayer et al. 1999, *Science* **286**:971-4), oder durch Mikrotubuli stabilisierende oder destabilisierende Agentien (z.B. Colchicin, Taxol, Etoposid, Vinblastin, Vincristin) ausgelöst werden (Schiff und Horwitz 1980, *Proc Natl Acad Sci* U S A **77**:1561-5).

Neben den Cyclin-abhängigen und den Aurora Kinasen spielen des weiteren die so genannten Polo-like Kinasen, eine kleine Familie von Serin/Threonin-Kinasen, eine wichtige Rolle bei der Regulation des eukaryontischen Zellzykluses. Bisher wurden die Polo-like Kinasen PLK-1, PLK-2, PLK-3 und PLK-4 in der Literatur beschrieben. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al. *1998, Genes Dev.* **12**:3777-87; Qian et al. 2001, *Mol Biol Cell*. **12**:1791-9). Die Injektion von PLK-1 Antikörpern führt zu einem G2 Arrest in nicht transformierten Zellen, während Tumorzellen in der Mitosephase arretieren (Lane und Nigg 1996, *J Cell Biol.* **135**:1701-13). Überexpression von PLK-1 konnte für verschiedene Tumorarten, wie nicht kleinzelliges Lungenkarzinom, Plattenepithelkarzinom, Brust- und kolorektales Karzinom (Wolf et al. 1997, *Oncogene* **14** :543 -549; Knecht et al. 1999, *Cancer Res.* **59**:2794 -2797; Wolf et al. 2000, *Pathol. Res. Pract.* **196**:753 -759; Takahashi et al. 2003, *Cancer Sci.* **94**:148-52) gezeigt werden. Daher stellt diese Klasse von Proteinen ebenfalls einen interessanten Angriffspunkt zur therapeutischen Intervention proliferativer Krankheiten dar (Liu and Erikson 2003, *Proc Natl Acad Sci U S A* **100**:5789-5794).
Die Resistenz vieler Tumorarten erfordert die Entwicklung neuer Arzneimittel zur Tumorbekämpfung.

Es ist die Aufgabe der vorliegenden Erfindung, neue Verbindungen mit antiproliferativer Wirkung bereitzustellen.

### Beschreibung der Erfindung

Die erfindungsgemäße Aufgabe wird durch die nachstehenden Verbindungen der Formel (I) gelöst.

Dementsprechend betrifft die Erfindung Dihydropteridinone der allgemeinen Formel (I) 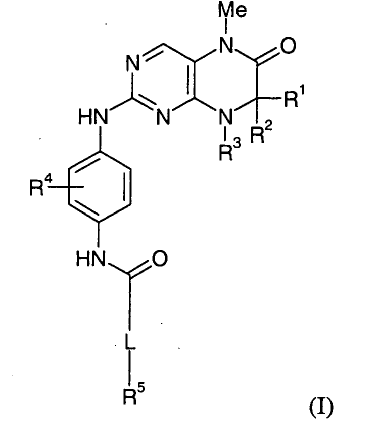
worin
- L: eine Einfachbindung, oder eine verbrückende zweibindige Gruppe ausgewählt aus der Gruppe bestehend aus C₁-C₆-Alkylen, C₂-C₆-Alkenylen, C₂-C₆₋Alkinylen, C₃-C₇-Cycloalkylen, C₁-C₄-Alkylen-C₆-C₁₀-Arylen-C₁-C₄-Alkylen, C₁₋C₄-Alkylen-C₆-C₁₀-Arylen, -O-, -O-C₁-C₆-Alkylen, -O-C₃-C₆-Alkenylen, -O-C₃-C₆₋Alkinylen, -O-C₃-C₇-Cycloalkylen, -O-C₁-C₄-Alkylen-C₆-C₁₀-Arylen-C₁-C₄₋Alkylen, -O-C₁-C₄-Alkylen-C₆-C₁₀-Arylen, -NR⁷- und -NR⁷-C₁-C₆-Alkylen, -NR⁷⁻C₃-C₆-Alkenylen, -NR⁷-C₃-C₆-Alkinylen, -NR⁷-C₃-C₇-Cycloalkylen, -NR⁷-C₁-C₄₋Alkylen-C₆-C₁₀-Arylen-C₁-C₄-Alkylen, -NR⁷-C₁-C₄-Alkylen-C₆-C₁₀-Arylen, welche gegebenenfalls substituiert sein kann durch einen oder mehrere Reste R⁹;
- R¹ und R²,: gleich oder verschieden, Wasserstoff, oder ein Rest ausgewählt aus der Gruppe bestehend aus C₁-C₆-Alkyl, C₂-C₆-Alkenyl und C₂-C₆-Alkinyl, der gegebenenfalls ein- oder mehrfach substituiert sein kann durch einen oder mehrere Reste R⁹, oder
- R¹ und R²: gemeinsam C₂-C₆-Alkylen, in welchem gegebenenfalls ein oder zwei Methylengruppen ersetzt sein können durch eine der Gruppen -O- oder -NR⁷-, und welches gegebenenfalls ein- oder mehrfach substituiert sein kann durch einen oder mehrere Reste R⁹;
- R³: Wasserstoff oder ein Rest ausgewählt aus C₁-C₈ -Alkyl, C₂-C₈-Alkenyl, C₂-C₈₋Alkinyl, C₃-C₈-Cycloalkyl und C₆-C₁₄-Aryl, der gegebenenfalls ein- oder mehrfach substituiert sein kann durch einen oder mehrere Reste R⁹; oder
- R³ und R²: oder R³ und R¹ gemeinsam C₂ -C₆-Alkylen, das gegebenenfalls ein- oder mehrfach substituiert sein kann durch einen oder mehrere Reste R⁹;
- R⁴: Wasserstoff, Halogen, CN, OH, -NR⁷R⁸ oder ein Rest ausgewählt aus der Gruppe bestehend aus C₁-C₆-Alkyl, C₁-C₆-Alkyloxy, C₂-C₆-Alkenyl, C₂-C₆-Alkenyloxy, C₂-C₆-Alkinyl und C₂-C₆-Alkinyloxy, der gegebenenfalls ein- oder mehrfach substituiert sein kann durch einen oder mehrere Reste R¹⁰;
- R⁵: Phenyl, dass gegebenenfalls ein oder mehrfach substituiert sein durch einen oder mehrere Reste R¹¹, oder
- R⁵: Phenyl, dass einfach substituiert ist durch einen Rest R⁶, oder
- R⁵: C₁-C₄-Alkyl, das gegebenenfalls ein oder mehrfach substituiert sein kann durch einen oder mehrere Reste R⁹, oder
- R⁶: -NR⁷R⁸ oder eine 5-10 gliedrige Heterocycloalkylgruppe, die ein-, zwei oder drei Heteroatome ausgewählt aus der Gruppe Stickstoff, Sauerstoff und Schwefel, bevorzugt Stickstoff oder Sauerstoff, enthalten kann und die gegebenenfalls ein- oder mehrfach substituiert sein kann durch einen oder mehrere der Reste R¹²;
- R⁷ und R⁸,: gleich oder verschieden, Wasserstoff oder C₁-C₆-Alkyl,
- R⁹: Halogen, C₁-C₄-Alkyl, C₁-C₄-Alkyloxy, CN, OH oder CF₃;
- R¹⁰: Halogen, OH, CN, =O, C₁-C₆-Alkyloxy, COOR⁷, NR⁷R⁸, CONR⁷R⁸, SO₂R⁷, CHF₂ oder CF₃;
- R¹¹: Halogen, OH, CN, C₁-C₄-Alkyl, C₁-C₄-Alkyloxy, COOR⁷, CONR⁷R⁸, SO₂R⁷, CHF₂, CF₃, C₆-C₁₀-Aryl oder C₁-C₆-Alkylen-C₆-C₁₀-Aryl;
- R¹²: Halogen, CF₃, C₁-C₆-Alkyl, C₁-C₆-Alkylen-C₆-C₁₀-Aryl, C₃-C₈-Cycloalkyl oder C₁-C₆-Alkylen-C₃-C₈-Cycloalkyl;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Bevorzugt sind Verbindungen der allgemeinen Formel (I), worin
- L: eine Einfachbindung, oder eine verbrückende zweibindige Gruppe ausgewählt aus der Gruppe bestehend aus C₁-C₆-Alkylen, C₂-C₆-Alkenylen, C₂-C₆-Alkinylen, C₃₋C₇-Cycloalkylen, C₁-C₄-Alkylen-C₆-C₁₀-Arylen-C₁-C₄-Alkylen, -O-, -O-C₁-C₄-Alkylen, -NR⁷- und -NR⁷-C₁-C₄-Alkylen, welche gegebenenfalls substituiert sein kann durch einen oder mehrere Reste R⁹;
- R¹ und R² ,: gleich oder verschieden, Wasserstoff, oder ein Rest ausgewählt aus der Gruppe bestehend aus C₁-C₆-Alkyl, C₂-C₆-Alkenyl und C₂-C₆-Alkinyl, der gegebenenfalls ein- oder mehrfach substituiert sein kann durch einen oder mehrere Reste R⁹, oder
- R¹ und R²: gemeinsam C₂-C₆-Alkylen, in welchem gegebenenfalls ein oder zwei Methylengruppen ersetzt sein können durch eine der Gruppen -O- oder -NR⁷-, und welches gegebenenfalls ein- oder mehrfach substituiert sein kann durch einen oder mehrere Reste R⁹;
- R³: Wasserstoff oder ein Rest ausgewählt aus C₁-C₈-Alkyl, C₂-C₈-Alkenyl, C₂-C₈₋Alkinyl, C₃-C₈-Cycloalkyl und C₆-C₁₄-Aryl, der gegebenenfalls ein- oder mehrfach substituiert sein kann durch einen oder mehrere Reste R⁹; oder
- R³ und R²: oder R³ und R¹ gemeinsam C₂-C₆-Alkylen, das gegebenenfalls ein- oder mehrfach substituiert sein kann durch einen oder mehrere Reste R⁹;
- R⁴: Wasserstoff, Halogen, CN, OH, -NR⁷R⁸ oder ein Rest ausgewählt aus der Gruppe bestehend aus C₁-C₆-Alkyl, C₁-C₆-Alkyloxy, C₂-C₆-Alkenyl, C₂-C₆-Alkenyloxy, C₂-C₆-Alkinyl und C₂-C₆-Alkinyloxy, der gegebenenfalls ein- oder mehrfach substituiert sein kann durch einen oder mehrere Reste R¹⁰;
- R⁵: Phenyl, dass gegebenenfalls ein oder mehrfach substituiert sein durch einen oder mehrere Reste R¹¹, oder
- R⁵: Phenyl, dass einfach substituiert ist durch einen Rest R⁶, oder
- R⁵: C₁-C₄-Alkyl, das gegebenenfalls ein oder mehrfach substituiert sein kann durch einen oder mehrere Reste R⁹, oder
- R⁶: eine 5-10 gliedrige Heterocycloalkylgruppe, die ein-, zwei oder drei Heteroatome ausgewählt aus der Gruppe Stickstoff, Sauerstoff und Schwefel, bevorzugt Stickstoff oder Sauerstoff, enthalten kann und die gegebenenfalls ein- oder mehrfach substituiert sein kann durch einen oder mehrere der Reste R¹²;
- R⁷ und R⁸,: gleich oder verschieden, Wasserstoff oder C₁-C₆-Alkyl,
- R⁹: Halogen, C₁-C₄-Alkyl, C₁-C₄-Alkyloxy, CN, OH oder CF₃;
- R¹⁰: Halogen, OH, CN, =O, C₁-C₆-Alkyloxy, COOR⁷, CONR⁷R⁸, SO₂R⁷, CHF₂ oder CF₃;
- R¹¹: Halogen, OH, CN, C₁-C₄-Alkyl, C₁-C₄-Alkyloxy, COOR⁷, CONR⁷R⁸, SO₂R⁷, CHF₂, CF₃, C₆-C₁₀-Aryl oder C₁-C₆-Alkylen-C₆-C₁₀-Aryl;
- R¹²: Halogen, CF₃, C₁-C₆-Alkyl, C₁-C₆-Alkylen-C₆-C₁₀-Aryl, C₃-C₈-Cycloalkyl oder C₁-C₆-Alkylen-C₃-C₈-Cycloalkyl;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Bevorzugt sind ferner Verbindungen der allgemeinen Formel (I), worin
- L: eine Einfachbindung, -O-, -O-C₁-C₃-Alkylen, -NR⁷-, -NR⁷-C₁-C₃-Alkylen oder C₁-C₄-Alkylen, welches gegebenenfalls substituiert sein kann durch einen oder mehrere Reste R⁹;
- R¹ und R² ,: gleich oder verschieden, Wasserstoff, oder ein Rest ausgewählt aus der Gruppe bestehend aus C₁-C₄-Alkyl, C₂-C₄-Alkenyl und C₂-C₄-Alkinyl, der gegebenenfalls ein- oder mehrfach substituiert sein kann durch einen oder mehrere Reste R⁹, oder
- R¹ und R²: gemeinsam C₂-C₄-Alkylen, das gegebenenfalls ein- oder mehrfach substituiert sein kann durch einen oder mehrere Reste R⁹;
- R³: Wasserstoff oder ein Rest ausgewählt aus C₁-C₆-Alkyl, C₃-C₇-Cycloalkyl und C₆₋C₁₀-Aryl, der gegebenenfalls ein- oder mehrfach substituiert sein kann durch einen oder mehrere Reste R⁹; oder
- R³ und R²: oder R³ und R¹ gemeinsam C₂-C₄-Alkylen, das gegebenenfalls ein- oder mehrfach substituiert sein kann durch einen oder mehrere Reste R⁹;
- R⁴: Wasserstoff, Fluor, Chlor, Brom, -NR⁷R⁸ oder ein Rest ausgewählt aus der Gruppe bestehend aus C₁-C₄-Alkyl, C₁-C₄-Alkyloxy, C₂-C₄-Alkenyloxy und C₂-C₄₋Alkinyloxy, der gegebenenfalls ein- oder mehrfach substituiert sein kann durch einen oder mehrere Reste R¹⁰;
- R⁵: Phenyl, dass gegebenenfalls ein- oder zweifach substituiert sein durch einen oder mehrere Reste R¹¹, oder
- R⁵: Phenyl, dass einfach substituiert ist durch einen Rest R⁶, oder
- R⁵: ein Rest ausgewählt aus der Gruppe bestehend aus Methyl, Ethyl, Propyl und Butyl, der gegebenenfalls ein oder zweifach substituiert sein kann durch einen oder mehrere Reste R⁹;
- R⁶: ein Heterocycloalkyl ausgewählt aus der Gruppe bestehend aus Piperidinyl, Piperazinyl, Pyrrolinyl, Pyrrolidinyl, Pyrazolinyl, Pyrazolidinyl, Imidazolinyl, Imidazolidinyl und Morpholinyl, das gegebenenfalls ein- oder mehrfach substituiert sein kann durch einen oder mehrere der Reste R¹²;
- R⁷ und R⁸,: gleich oder verschieden, Wasserstoff oder C₁-C₄-Alkyl,
- R⁹: Halogen, C₁-C₄-Alkyl, C₁-C₄-Alkyloxy, CN, OH oder CF₃;
- R¹⁰: Halogen, OH, =O, C₁-C₄-Alkyloxy oder CF₃;
- R¹¹: Halogen, OH, CN, C₁-C₄-Alkyl, C₁-C₄-Alkyloxy, COOR⁷, CF₃, oder C₁-C₄₋Alkylen-Phenyl;
- R¹²: C₁-C₄-Alkyl, C₁-C₄-Alkylen-Phenyl, C₃-C₆-Cycloalkyl oder C₁-C₄-Alkylen-C₃-C₆₋Cycloalkyl;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Bevorzugt sind ferner Verbindungen der allgemeinen Formel (I) ,
worin
- L: eine Einfachbindung, C₁-C₄-Alkylen, -O-, -O-C₁-C₃-Alkylen, -NH- oder -NH-C₁-C₃-Alkylen;
- R¹ und R²,: gleich oder verschieden, Wasserstoff, oder ein Rest ausgewählt aus der Gruppe bestehend aus C₁-C₄-Alkyl, C₂-C₄-Alkenyl und C₂-C₄-Alkinyl, der gegebenenfalls ein- oder zweifach substituiert sein kann durch einen Rest ausgewählt aus der Gruppe bestehend aus Fluor, Chlor, OH und CF₃; oder
- R³: Wasserstoff oder ein Rest ausgewählt aus C₁-C₆-Alkyl, C₃-C₇-Cycloalkyl und C₆₋C₁₀-Aryl, der gegebenenfalls ein- oder zweifach substituiert sein kann durch einen Rest ausgewählt aus der Gruppe bestehend aus Fluor, Chlor, Methyl, Ethyl, OH, Methyloxy, Ethyloxy und CF₃;
- R⁴: Wasserstoff, Fluor, Chlor, Brom, -NR⁷R⁸ oder ein Rest ausgewählt aus der Gruppe bestehend aus C₁-C₄-Alkyl, C₁-C₄-Alkyloxy, C₂-C₄-Alkenyloxy und C₂-C₄₋Alkinyloxy, der gegebenenfalls ein- oder zweifach substituiert sein kann durch einen Rest ausgewählt aus der Gruppe bestehend aus Fluor, Chlor, OH, Methoxy, Ethoxy und CF₃;
- R⁵: Phenyl, das gegebenenfalls ein- oder zweifach substituiert sein kann durch einen Rest ausgewählt aus der Gruppe bestehend aus Methyl, Ethyl, OH, Fluor, Chlor, CF₃, COOH, COOMethyl oder COOEthyl, oder
- R⁵: Phenyl, das einfach substituiert ist durch ein Heterocycloalkyl ausgewählt aus der Gruppe bestehend aus Piperidinyl, Piperazinyl, Pyrrolinyl, Pyrrolidinyl, Pyrazolinyl, Pyrazolidinyl, Imidazolinyl, Imidazolidinyl und Morpholinyl, der ein- oder zweifach substituiert sein kann durch Methyl, Ethyl, Benzyl, Phenethyl, Cyclopropyl oder Cyclopropylmethyl, oder
- R⁵: ein Rest ausgewählt aus der Gruppe bestehend aus Methyl, Ethyl, Propyl und Butyl, der gegebenenfalls ein oder zweifach substituiert sein kann durch einen oder mehrere Reste ausgewählt aus der Gruppe Fluor, Chlor, OH und CF₃;
- R⁷ und R⁸,: gleich oder verschieden, Wasserstoff, Methyl oder Ethyl, bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Bevorzugt sind ferner Verbindungen der allgemeinen Formel (I) ,
worin
- eine Einfachbindung, -CH₂-, -CH₂-CH₂-, -O-, -O-CH₂-, -O-CH₂-CH₂-, -NH-, -NH-CH₂- oder -NH-CH₂-CH₂- ;
- R¹ und R²,: gleich oder verschieden Wasserstoff oder ein Rest ausgewählt aus der Gruppe bestehend aus Methyl, Ethyl, Propyl, Butyl, Allyl und Propargyl, der gegebenenfalls ein- oder zweifach substituiert sein kann durch einen Rest ausgewählt aus der Gruppe bestehend aus Fluor, Chlor und CF₃;
- R³: Wasserstoff oder ein Rest ausgewählt aus der Gruppe bestehend aus Methyl, Ethyl, Propyl, Butyl, Pentyl, Cyclopropyl, Cyclopentyl, Cyclohexyl und Phenyl, der gegebenenfalls ein- oder zweifach substituiert sein kann durch einen Rest ausgewählt aus der Gruppe bestehend aus Fluor, Chlor, Methyl, Ethyl, Methyloxy, Ethyloxy und CF₃;
- R⁴: Wasserstoff, Methyl, Ethyl, Propyl, Methyloxy, Ethyloxy oder Propyloxy;
- R⁵: Phenyl, das gegebenenfalls ein- oder zweifach substituiert sein kann durch einen Rest ausgewählt aus der Gruppe bestehend aus Methyl, OH, Fluor, CF₃, COOH, COOMethyl oder COOEthyl, oder
- R⁵: Phenyl, das einfach substituiert ist durch ein Heterocycloalkyl ausgewählt aus der Gruppe bestehend aus Piperidinyl, Piperazinyl, Pyrrolidinyl und Morpholinyl, der ein- oder zweifach substituiert sein kann durch Methyl, Ethyl, Benzyl, Phenethyl, Cyclopropyl oder Cyclopropylmethyl;
- R⁵: ein Rest ausgewählt aus der Gruppe bestehend aus Methyl, Ethyl, Propyl und Butyl, der gegebenenfalls ein oder zweifach substituiert sein kann durch einen oder mehrere Reste ausgewählt aus der Gruppe Fluor, Chlor und CF₃;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I) , worin
- L: eine Einfachbindung, oder eine verbrückende zweibindige Gruppe ausgewählt aus der Gruppe bestehend aus -CH₂-, -CH₂-CH₂-, -O-, -O-CH₂-, -O-CH₂-CH₂-, -NH-, -NH-CH₂- oder -NH-CH₂-CH₂-, bevorzugt eine Einfachbindung oder eine Gruppe ausgewählt aus -CH₂-, -CH₂-CH₂-, -O-, -O-CH₂-, -NH- oder -NH-CH₂-, bedeuten
und die Reste R¹, R², R³, R⁴ und R⁵ und R⁹ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.
Bei unsymmetrischen Gruppen L, können die genannten verbrückenden, zweibindigen Gruppen in zwei verschiedenen Ausrichtungen verknüpft sein. Erfindungsgemäß bevorzugt sind diejenigen Verbindungen der Formel (I), bei denen im Falle einer unsymmetrischen Gruppe L, das Heteroatom der Gruppe L mit der Carbonylfunktion der Verbindung der Formel (I) verknüpft ist.

Erfindungsgemäß von besonderer Bedeutung sind ferner Verbindungen der allgemeinen Formel (I) , worin
- L: -NH-, -NH-CH₂- oder -NH-CH₂-CH₂- bedeutet,
- R⁴: Wasserstoff ist
und die Reste R¹, R², R³ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind ferner Verbindungen der allgemeinen Formel (I) , worin L -O-, -O-CH₂- oder -O-CH₂-CH₂- bedeutet und die Reste R¹, R², R³, R⁴ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I) , worin R¹ Wasserstoff, Methyl Ethyl, Allyl oder Propargyl, bevorzugt Wasserstoff oder Methyl, besonders bevorzugt Wasserstoff bedeuten und die Reste L, R², R³, R⁴ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I) , worin R² Wasserstoff, Methyl, Ethyl, Allyl oder Propargyl, bevorzugt Wasserstof, Methyl oder Ethyl, besonders bevorzugt Methyl oder Ethyl bedeuten und die Reste L, R¹, R³, R⁴ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I) , worin
- R³: Methyl, Ethyl, Propyl, Butyl, Pentyl, Cyclopropyl, Cyclopentyl oder Cyclohexyl bevorzugt Propyl, Butyl, Pentyl, Cyclopentyl oder Cyclohexyl, besonders bevorzugt Propyl, Butyl, Pentyl, Cyclopentyl oder Cyclohexyl, bedeuten, wobei Propyl, Pentyl, Cyclopentyl oder Cyclohexyl, insbesondere Cyclypentyl und Cyclohexyl von besonderer Bedeutung sind,
und die Reste L, R¹, R², R⁴ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I) , worin R³ Phenyl bedeutet, das gegebenenfalls ein- oder zweifach, bevorzugt einfach substituiert sein kann durch Fluor, Chlor, Methyl, Ethyl, Methyloxy, Ethyloxy und CF₃, bevorzugt Phenyl, das gegebenenfalls ein- oder zweifach, bevorzugt einfach substituiert sein kann durch Fluor, Methyl oder Methyloxy, und die Reste L, R¹, R², R⁴ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I) , worin
- R⁴: Wasserstoff, Methyl, Ethyl, Methyloxy oder Ethyloxy, bevorzugt Wasserstoff, Methyl oder Methyloxy, besonders bevorzugt Wasserstoff oder Methyloxy bedeuten,
und die Reste L, R¹, R², R³ und R⁵ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I), worin
- R⁵: Phenyl, das gegebenenfalls ein- oder zweifach substituiert sein kann durch einen Rest ausgewählt aus der Gruppe bestehend aus Methyl, OH, Fluor, CF₃, COOH, COOMethyl oder COOEthyl, bevorzugt ausgewählt aus der Gruppe bestehend aus Fluor, CF₃, COOH, COOMethyl oder COOEthyl, bedeutet
und die Reste L, R¹, R², R³ und R⁴ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I), worin
- R⁵: Phenyl, das einfach substituiert ist durch ein Heterocycloalkyl ausgewählt aus der Gruppe bestehend aus Piperidinyl, Piperazinyl, Pyrrolidinyl und Morpholinyl, das ein- oder zweifach substituiert sein kann durch Methyl, Ethyl, Benzyl, Phenethyl, Cyclopropyl oder Cyclopropylmethyl bedeutet
und die Reste L, R¹, R², R³ und R⁴ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I), worin
- R⁵: Phenyl, das einfach substituiert ist durch ein Heterocycloalkyl ausgewählt aus der Gruppe bestehend aus Piperidinyl, Piperazinyl und Morpholinyl, das ein- oder zweifach substituiert sein kann durch Methyl, Ethyl, Benzyl oder Cyclopropylmethyl, bevorzugt Methyl oder Ethyl, bedeutet
und die Reste L, R¹, R², R³ und R⁴ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I), worin
- R⁵: ein Rest ausgewählt aus der Gruppe bestehend aus Methyl, Ethyl, Propyl und Butyl, der gegebenenfalls ein oder zweifach substituiert sein kann durch einen oder mehrere Reste ausgewählt aus der Gruppe Fluor, Chlor und CF₃, bedeutet
und die Reste L, R¹, R², R³ und R⁴ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Erfindungsgemäß von besonderer Bedeutung sind Verbindungen der allgemeinen Formel (I), worin R⁵ ein Rest ausgewählt aus der Gruppe bestehend aus Methyl, Ethyl, Propyl und Butyl, bedeutet und die Reste L, R¹, R², R³ und R⁴ eine der vor- oder nachstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Als Alkylgruppen sowie Alkylgruppen, welche Bestandteil anderer Reste sind, werden verzweigte und unverzweigte Alkylgruppen mit 1 bis 8 Kohlenstoffatomen bevorzugt 1 - 6, besonders bevorzugt 1-4 Kohlenstoffatomen bezeichnet. Beispielsweise werden genannt: Methyl, Ethyl, Propyl, Butyl, Pentyl, Hexyl, Heptyl und Octyl. Sofern nicht anders genannt, sind von den vorstehend genannten Bezeichnungen Propyl, Butyl, Pentyl, Hexyl, Heptyl und Octyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.
In den vorstehend genannten Alkylgruppen können sofern nicht gegenteilig oder ergänzend definiert gegebenenfalls ein oder mehrere Wasserstoffatome durch andere Reste ersetzt sein. Beispielsweise können diese Alkylgruppen durch Fluor substituiert sein. Es können gegebenenfalls auch alle Wasserstoffatome der Alkylgruppe ersetzt sein.

Als Alkyloxygruppen, gegebenenfalls auch Alkoxygruppen oder -O-Alkylgruppen genannt, werden vorstehend genannte Alkylgruppen bezeichnet, die über eine Sauerstoffbrücke verknüpft sind. Beispielsweise werden genannt: Methyloxy, Ethyloxy, Propyloxy, Butyloxy, Pentyloxy, Hexyloxy, Heptyloxy und Octyloxy, die gegebenenfalls auch als Methoxy, Ethoxy, Propxy etc. bezeichnet werden.

Als Alkylenbrücke oder Alkylengruppe werden, soweit nicht anders angegeben,
verzweigte und unverzweigte Alkylgruppen mit 1 bis 6 Kohlenstoffatomen, beispielsweise Methylen, Etyhlen, Propylen, Isopropylen, n-Butylen, iso-Butyl, sec. Butyl und tert.-Butyl etc. Brücken bezeichnet. Besonders bevorzugt sind Methylen, Etyhlen, Propylen- und Butylen-Brücken. In den genannten Alkylenbrücken können sofern nicht gegenteilig oder ergänzend definiert gegebenenfalls 1 bis 2 C-Atome durch ein oder mehrere Heteroatome ausgewählt aus der Gruppe Sauerstoff, Stickstoff oder Schwefel ersetzt sein

Als Alkenylgruppen (auch soweit sie Bestandteil anderer Reste sind) werden verzweigte und unverzweigte Alkylengruppen mit 2 bis 8 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 3-Butenyl, 1-Methyl-1-Propenyl, 1-Methyl-2-Propenyl, 2-Methyl-1-Propenyl, 2-Methyl-2-Propenyl und 1-Ethyl-1-Ethenyl.

In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor substituiert sein. Es können gegebenenfalls auch alle Wasserstoffatome der Alkenylgruppe ersetzt sein.

Als Alkenyloxygruppen, gegebenenfalls auch Alkenoxygruppen oder -O-Alkenylgruppen genannt, werden vorstehend genannte Alkenylgruppen bezeichnet, die über eine Sauerstoffbrücke verknüpft sind. Beispielsweise werden genannt: Ethylenoxy, Propylenoxy, Butylenoxy.

Als Alkenylengruppen (auch soweit sie Bestandteil anderer Reste sind) werden verzweigte und unverzweigte, verbrückende Alkylengruppen mit 2 bis 6 Kohlenstoffatomen, bevorzugt 2 - 4 Kohlenstoffatomen, besonders bevorzugt 2 - 3 Kohlenstoffatomen betrachtet, soweit sie mindestens eine Doppelbindung aufweisen. Beispielsweise werden genannt: Ethenylen, Propenylen etc. Sofern nicht anders genannt, sind von den vorstehend genannten Bezeichnungen Propenylen, Butenylen etc. sämtliche der möglichen isomeren Formen umfaßt.

Als Alkinylgruppen (auch soweit sie Bestandteil anderer Reste sind) werden verzweigte und unverzweigte Alkinylgruppen mit 2 bis 8 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.
In den vorstehend genannten Alkinylgruppen können, soweit nicht anders beschrieben, gegebenenfalls ein oder mehrere Wasserstoffatome durch andere Reste ersetzt sein. Beispielsweise können diese Alkinylgruppen Fluor substituiert sein. Es können gegebenenfalls auch alle Wasserstoffatome der Alkinylgruppe ersetzt sein.

Als Alkinyloxygruppen, gegebenenfalls auch Alkinoxygruppen oder -O-Alkinylgruppen genannt, werden vorstehend genannte Alkinylgruppen bezeichnet, die über eine Sauerstoffbrücke verknüpft sind.

Als Alkinylengruppen (auch soweit sie Bestandteil anderer Reste sind) werden verzweigte und unverzweigte, verbrückende Alkinylgruppen mit 2 bis 6 Kohlenstoffatomen bezeichnet, soweit sie mindestens eine Dreifachbindung aufweisen, beispielsweise Ethinylen, Propargylen etc. In den vorstehend genannten Alkinylengruppen können, soweit nicht anders beschrieben, gegebenenfalls ein oder mehrere Wasserstoffatome durch andere Reste ersetzt sein. Sofern nicht anders genannt, sind von den vorstehend genannten Bezeichnungen Propargylen etc. sämtliche der möglichen isomeren Formen umfaßt.

Der Begriff Aryl steht für ein aromatisches Ringsystem mit 6 bis 14 Kohlenstoffatomen, vorzugsweise 6 oder 10 Kohlenstoffatomen, bevorzugt Phenyl oder Naphthyl, besonders bevorzugt Phenyl.

Als Cycloalkylgruppen werden Cycloalkylreste mit 3 - 8 Kohlenstoffatomen beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet. Bis auf Cylopropyl und Cyclobutyl, können die genannten Cycloalklgruppen gegebenenfalls auch partiell ungesättigt sein, das bedeutet wenigstens eine Doppelbindung enthalten wie beispielsweise Cyclohexen. Die Bezeichnung Cylcoalkylengruppe steht für verbrückende, zweibindige Cycloalkylgruppen.

"=O" bedeutet ein über eine Doppelbindung verknüpftes Sauerstoffatom.

Als 5-10 gliedrige Heterocycloalkylgruppe, die ein-, zwei oder drei Heteroatome ausgewählt aus der Gruppe Stickstoff, Sauerstoff und Schwefel, bevorzugt Stickstoff oder Sauerstoff, enthalten kann, werden, soweit in den Definitionen nicht anders beschrieben, beispielsweise genannt Tetrahydrofuranyl, Tetrahydrofuranonyl, γ-Butyrolactonyl, α-Pyranyl, γ-Pyranyl, Dioxolanyl, Tetrahydropyranyl, Dioxanyl, Dihydrothiophenyl, Thiolanyl, Dithiolanyl, Pyrrolinyl, Pyrrolidinyl, Pyrazolinyl, Pyrazolidinyl, Imidazolinyl, Imidazolidinyl, Piperidinyl, Piperazinyl, Morpholinyl, Thiomorpholinyl, Diazepanyl, Oxazinyl, Tetrahydrooxazinyl, Pyrazolidinyl genannt, vorzugsweise Morpholinyl, Pyrrolidinyl, Piperidinyl oder Piperazinyl, genannt.

Als Halogen wird im allgemeinen Fluor, Chlor, Brom oder Jod bezeichnet, vorzugsweise Fluor , Chlor oder Brom, besonders bevorzugt Fluor oder Chlor.

Die erfindungsgemäßen Verbindungen können in Form der einzelnen optischen Isomeren, Mischungen der einzelnen Enantiomeren, Diastereomeren oder Racemate, in Form der Tautomere sowie in Form der freien Basen oder der entsprechenden Säureadditionssalze mit pharmakologisch unbedenklichen Säuren vorliegen. Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Von den gegebenenfalls existierenden Enantiomeren und Diastereomeren Verbindungen der allgemeinen Formel (I) sind erfindungsgemäß diejenigen optischen Isomere bevorzugt, die am die beiden Reste R1 und R2 tragenden Kohlenstoffzentrum R-Konfiguration aufweisen.

Der Rest R⁴ kann, sofern er nicht Wasserstoff bedeutet, in den Verbindungen der allgemeinen Formel (I) bezüglich der an das Pteridinon-gerüst verknüpften NH-Gruppe ortho- oder meta-ständig verknüpft sein. Besonders bevorzugt sind diejenigen Verbindungen der allgemeinen Formel (I) in denen R⁴ zur genannten NH-Gruppe orthoständig konfiguriert ist. Diese bevorzugten Verbindungen sind gekennzeichnet durch die allgemeine Formel (I') 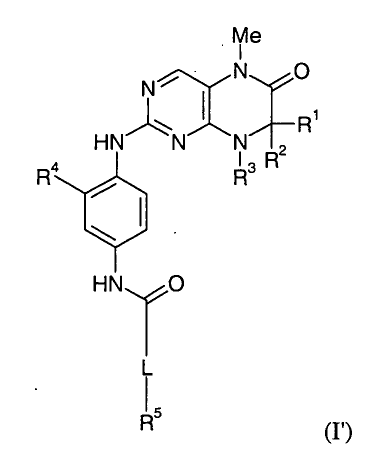
wobei die Reste L, R¹, R², R³, R⁴ und R⁵ die vorstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

Die Herstellung der erfindungsgemäßen Verbindungen kann nach den im folgenden beschriebenen Syntheseverfahren A erfolgen, wobei die Substituenten der allgemeinen Formeln (A1) bis (A9) die zuvor genannten Bedeutungen haben. Dieses Verfahren ist als Erläuterung der Erfindung zu verstehen ohne selbige auf deren Gegenstand zu beschränken.

### Verfahren A

### Stufe 1A

Eine Verbindung der Formel (A1) wird mit einer Verbindung der Formel (A2) zu einer Verbindung der Formel (A3) umgesetzt (Schema 1A). Diese Reaktion kann gemäß WO 0043369 oder WO 0043372 durchgeführt werden. Verbindung (A1) ist kommerziell, beispielsweise bei City Chemical LLC, 139 Allings Crossing Road, West Haven, CT, 06516, USA, erhältlich. Verbindung (A2) kann nach literaturbekannten Vorschriften (*a*) F. Effenberger, U. Burkhart, J. Willfahrt *Liebigs Ann. Chem.* **1986**, 314-333. b) T. Fukuyama, C.-K. Jow, M. Cheung, *Tetrahedron Lett.* **1995**, *36*, 6373-6374. c) R. K. Olsen, *J*. *Org. Chem*. **1970**, *35*, 1912-1915. d) F.E. Dutton, B.H. Byung *Tetrahedron Lett.* **1998**, *30,* 5313-5316. e) J. M. Ranajuhi, M. M. Joullie *Synth. Commun.* **1996**, *26*, 1379-1384 hergestellt werden. 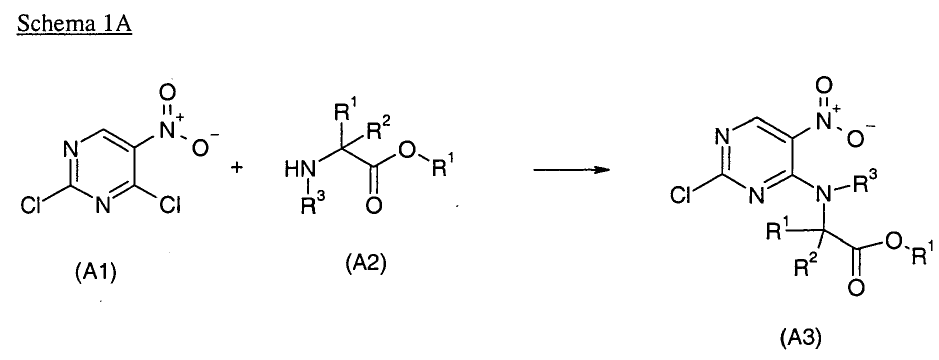 In Stufe 1A werden 1 Äquivalent der Verbindung (A1) und 1 bis 1,5 Äquivalente, bevorzugt 1,1 Äquivalente einer Base, vorzugsweise Kaliumcarbonat, Kaliumhydrogencarbonat, Natriumcarbonat oder Natriumhydrogencarbonat, Calciumcarbonat, besonders bevorzugt Kaliumcarbonat, in einem Verdünnungsmittel gegebenfalls mit Wasser gemischt, beispielsweise Aceton, Tetrahydrofuran, Diethylether, Cyclohexan, Methylcyclohexan, Petrolether oder Dioxan, vorzugsweise Cyclohexan oder Diethylether, gerührt.

Bei einer Temperatur von 0 bis 15 °C, bevorzugt 5 bis 10 °C, wird 1 Äquivalent einer Aminosäure der Formel (A2) gelöst in einem organischen Lösungsmittel, beispielsweise Aceton, Tetrahydrofuran, Diethylether, Cyclohexan oder Dioxan, zugetropft. Das Reaktionsgemisch wird unter Rühren auf eine Temperatur von 18°C bis 30 °C, vorzugsweise etwa 22°C, erwärmt und anschließend weitere 10 bis 24 Stunden, vorzugsweise etwa 12 Stunden, weitergerührt. Danach wird das Verdünnungsmittel abdestilliert, der Rückstand mit Wasser versetzt und das Gemisch zwei bis dreimal eines organischen Lösungsmittels beispielsweise, Diethylether oder Ethylacetat, vorzugsweise Ethylacetat extrahiert. Die vereinigten organischen Extrakte werden getrocknet und das Lösungsmittel abdestilliert. Der Rückstand (Verbindung A3) kann ohne vorherige Aufreinigung in Stufe 2 eingesetzt werden.

### Stufe 2A

Die in Stufe 1A erhaltene Verbindung (A3) wird an der Nitrogruppe reduziert und zur Verbindung der Formel (A4) zyklisiert (Schema 2A). 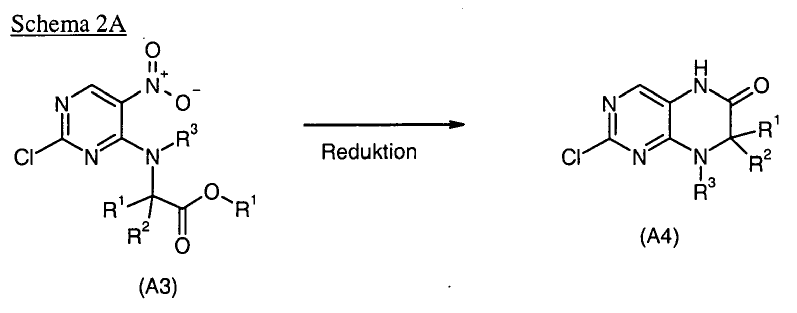 In Stufe 2A werden 1 Äquivalent der Nitroverbindung (A3) in einer Säure gelöst, vorzugsweise Eisessig, Ameisensäure oder wässrige Salzsäure, bevorzugt Eisessig, und auf 50 bis 70 °C, vorzugsweise etwa 60 °C, erwärmt. Anschließend wird ein Reduktionsmittel, beispielsweise Zink, Zinn, oder Eisen, bevorzugt Eisenpulver, bis zum Abschluß der exothermen Reaktion hinzugefügt und 0,2 bis 2 Stunden, vorzugsweise 0,5 Stunden, bei 100 bis 125 °C, vorzugsweise bei etwa 115 °C, gerührt. Nach Abkühlung auf Raumtemperatur wird das Eisensalz abfiltriert und das Lösungsmittel abdestilliert. Der Rückstand wird in einem Lösungsmittel oder Lösungsmittelgemisch, beispielsweise Ethylacetat oder Dichlormethan/ Methanol 9/1 und halbgesättigte NaCl-Lösung, aufgenommen und beispielsweise über Kieselgur filtriert. Die organische Phase wird getrocknet und eingeengt. Der Rückstand (Verbindung (A4)) kann chromatographisch oder mittels Kristallisation gereinigt oder als Rohprodukt in Stufe 3A der Synthese eingesetzt werden.

### Stufe 3A

Die in Stufe 2A erhaltene Verbindung (A4) kann durch elektrophile Substitution gemäß Schema 3A zur Verbindung der Formel (A5) umgesetzt werden. 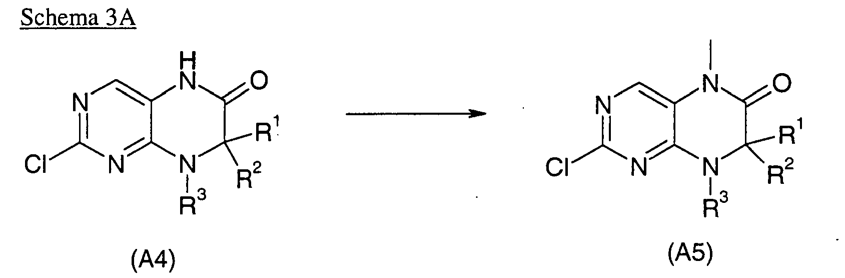 In Stufe 3A werden 1 Äquivalent des Amides der Formel (A4) in einem organischen Lösungsmittel, beispielsweise Dimethylformamid oder Dimethylacetamid, vorzugsweise Dimethylacetamid, gelöst und auf etwa -5 bis 5 °C, vorzugsweise 0°C, abgekühlt. Anschließend wird 0,9 bis 1,3 Äquivalente Natriumhydrid und 0,9 bis 1,3 Äquivalente eines Methylierungsreagenzes, beispielsweise Methyliodid zugegeben. Das Reaktionsgemisch wird 0,1-3 Stunden, vorzugsweise etwa 1 Stunde, bei etwa 0 bis 10 °C, vorzugsweise bei etwa 5 °C, gerührt und kann gegebenenfalls weitere 12 Stunden bei diesem Temperaturbereich stehengelassen werden. Die Reaktionsmischung wird auf Eiswasser gegossen und der Niederschlag isoliert. Der Rückstand (Verbindung (A5)) kann chromatographisch, vorzugsweise über Kieselgel, oder mittels Kristallisation gereinigt oder als Rohprodukt in Stufe 4A der Synthese eingesetzt werden.

### Stufe 4A

Die Aminierung der in Stufe 3A erhaltenen Verbindung (A5) zur Verbindung der Formel (I) (Schema 4A) kann nach den literaturbekannten Methoden der Varianten 4.1A (a) M.P.V. Boarland, J.F.W. McOmie *J. Chem. Soc.* 1951, 1218-1221; b) F. H. S. Curd, F. C. Rose *J. Chem. Soc.* 1946, 343-348 oder 4.2A (a) Banks *J. Am. Chem. Soc.* 1944, *66*, 1131; b) Ghosh and Dolly *J. Indian Chem. Soc.* 1981, *58*, 512-513. c) N. P. Reddy and M. Tanaka *Tetrahedron Lett.* 1997, *38*, 4807- 4810 durchgeführt werden. 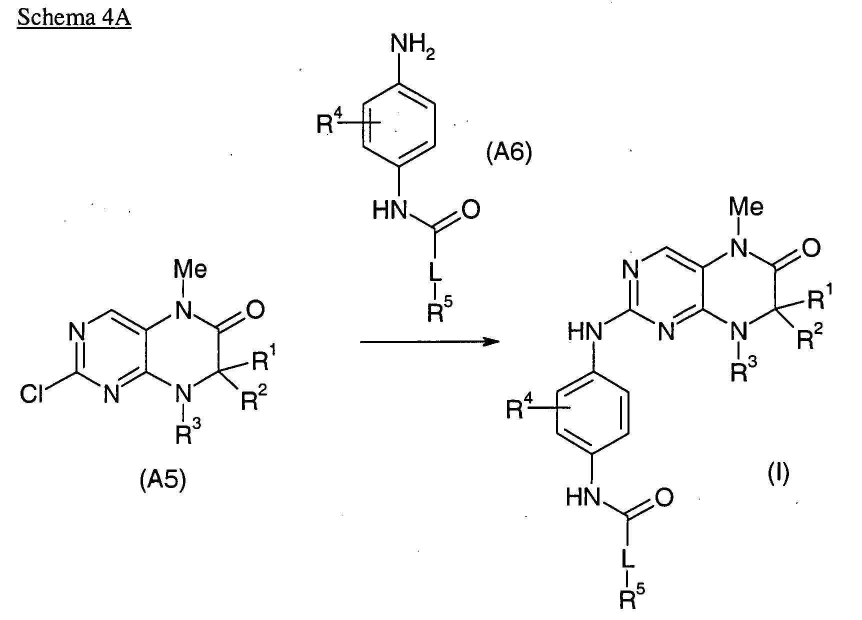 Beispielsweise werden in Variante 4.1A 1 Äquivalent der Verbindung (A5) und 1 bis 3 Äquivalente, vorzugsweise etwa 1 Äquivalent der Verbindung (A6) ohne Lösungsmittel oder einem organischen Lösungsmittel wie beispielsweise Sulfolan, Dimethylformamid, Dimethylacetamid, Toluol, N-Methylpyrrolidon, Dimethylsulfoxid, oder Dioxan, bevorzugt Sulfolan über 0,1 bis 4 Stunden, vorzugsweise 1 Stunde, bei 100 bis 220 °C, vorzugsweise bei etwa 160 °C erhitzt. Nach dem Abkühlen wird durch Zugabe von org. Lösungsmitteln oder Lösungsmittelgemischen bespielsweise Diethylether/Methanol, Ethylacetat, Methylenchlorid, oder Diethylether, vorzugsweise Diethylether/Methanol 9/1, das Produkt (A9) kristallisiert oder chromatographisch gereinigt.

Beispielsweise wird in Variante 4.2A 1 Äquivalent der Verbindung (A5) und 1 bis 3 Äquivalente der Verbindung (A6) mit Säure, beispielsweise 1-10 Äquivalente 10-38%ige Salzsäure und/oder einem Alkohol beispielsweise Ethanol, Propanol, Dioxan, Butanol, bevorzugt Ethanol unter Rückfluss 1 bis 48 Stunden, vorzugsweise etwa 5 Stunden, gerührt.

Das ausgefallene Produkt der Formel (I) wird abfiltriert und gegebenenfalls mit Wasser gewaschen, getrocknet und aus einem geeigneten org. Lösungsmittel kristallisiert.

Wie in Schema 4A ersichtlich, sind für die Synthese der erfindungsgemäßen Verbindungen der allgemeinen Formel (I) die Verbindungen der Formel (A5) von zentraler Bedeutung. Dementsprechend betrifft die vorliegende Erfindung ferner Zwischenverbindungen der allgemeinen Formel (A5) 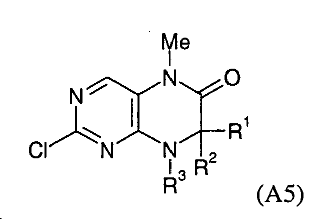
worin die Reste R¹, R² und R³ die vorstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

Analog zu den beschriebenen Verfahren werden die Produkte der Formel (A7) erhalten, die nach weiteren Umsetzung in die Produkte der Formel (I) überführt werden (siehe Stufe 5A). 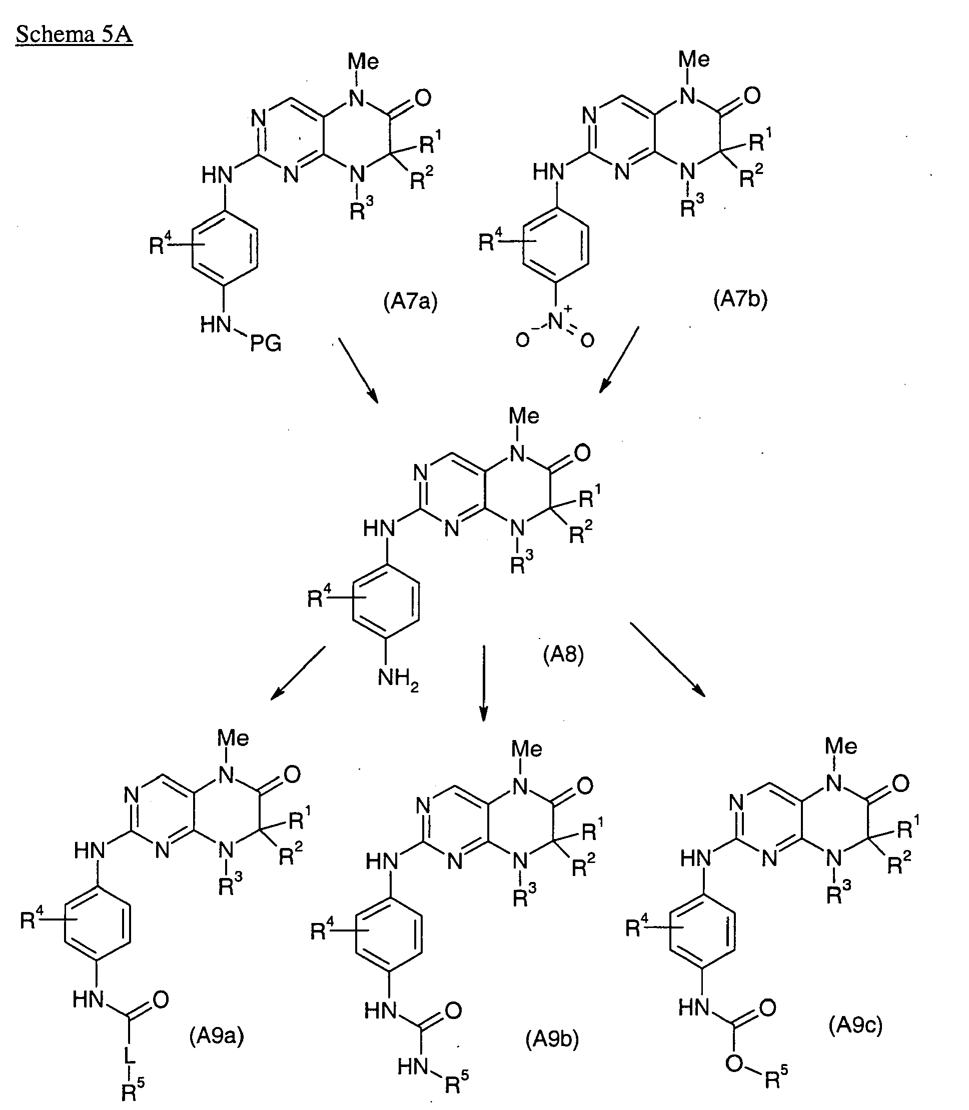 Als Gruppe PG, wie sie in vorstehendem Schema bei den Verbindungen (A7a) genannt ist, kommen im Stand der Technik bekannte Aminoschutzgruppen in Betracht. Geeignete Methoden zur Abspaltung der Gruppe PG und damit zur Überführung in die Verbindungen der Formel (A8) sind im Stand der Technik bekannt (vgl. T.W. Greene, "Protective Groups in Organic Synthesis", 2nd Edition).

Die in Schema 5A genannten Verbindungen der Formeln (A9a), (A9b) und (A9c) sind spezifische Vertreter der erfindungsgemäßen Verbindungen der Formel (I). In den Verbindungen der Formel (A9a) steht L dabei nicht für eine Gruppe L, die an den Carbonylkohlenstoff über ein -NH- oder -O- Brücke verknüpft ist. Diese Verbindungen sind vielmehr durch die Formeln (A9b) und (A9c) widergegeben.

### Stufe 5A

Nachfolgend der Aminierung in Stufe 4A können die Produkte der Formel (A9) auch über eine Abspaltung einer beispielsweise säure- oder basenlabilen Gruppe von Verbindungen des Types (A7) oder durch Reduktion einer Nitrogruppe zum Amin (A8) und anschliessende Umsetzung zu Amiden (A9a), Urethanen (A9b) oder Harnstoffen (A9c) erhalten werden (vgl. Schema 5A).

### Variante 5.1A:

Beispielsweise werden 1 Äquivalent einer Verbindung (A7a) mit einer säurelabilen Schutzgruppe beispielsweise tert-Butyloxycarbonyl mit 1- 50 Äquivalenten einer Säure bevorzugt HCl oder Trifluoressigsäure in einem organischem Lösungsmittel z.B Methylenchlorid, Ether, Dioxan, Tetrahydrofuran, bevorzugt Methylenchlorid versetzt und bei 20-100°C , bevorzugt 20°C für 1 bis 24h gerührt. Die Reaktionsmischung wird beispielsweise über Kieselgel getrennt oder durch geeignete Kristallisation erhalten.

### Variante 5.2B:

Beispielsweise werden 1 Äquivalent einer Verbindung (A7b) in einem Lösungsmittel z.B. Methanol, Ethanol, THF, Essigester, Wasser gelöst und mit 0,001 bis 0,1 Äquivalent Pd/C (10%) versetzt und mit Wasserstoff für 1-24 h hydriert. Nach Filtration des Katalysators erhält man das Produkt (A8) welches eventuell durch Kieselgelchromatographie oder durch eine geeignete Kristallisation aufgereinigt wird.

### Herstellung der Amide (A9a):

Beispielsweise wird 1 Äquivalent der Verbindung (A8) mit 1 Äquivalent eines Aktivierungsreagenzes, beispielesweise O-Benzotriazolyl-N,N,N',N'-tetramethyluroniumtetrafluoroborat (TBTU) und einer Base, beispielsweise etwa 1,5 Äquivalente, Diisopropylethylamin (DIPEA) in einem organischen Verdünnungsmittels, beispielsweise Dichlormethan, Tetrahydrofuran, Dimethylformamid, N-Methylpyrrolidion, Dimethylacetamid, vorzugsweise Dichlormethan oder Dimethylformamid, gelöst. Nach Zugabe von 1 Äquivalent des Amins (A10) wird das Reaktionsgemisch 0,1 bis 24 Stunden, vorzugsweise etwa 2 Stunden bei 20°C bis 100°C gerührt. Über beispielsweise Kristallisation oder chromatographische Reinigung wird das Produkt der Formel (A9a) erhalten.

### Herstellung der Harnstoffe (A9b):

Die in Schema 5A genannten Verbindungen A9b stellen Verbindungen dar, in denen L für eine NH Gruppe steht. Die nachstehend beschriebenen Vorgehensweisen sind, wie für den Fachmann klar ersichtlich, auch anwendbar, wenn L nicht lediglich NH, sondern beispielsweise -NH-Alkylen bedeutet.

### Variante A:

1 Äquivalent Amin (A8) wird in einem organischen Lösungsmittel beispielsweise Dichlormethan, THF, Dimethylformamid und einer Base beispielsweise Pyridin, Triethylamin, Disopropylethylamin gelöst und mit 1-2 Äquivalenten Chlorameisensäure-4-nitrophenylester, bevorzugt 1 Äquivalent versetzt. Nach 1-24 h, bevorzugt 2-5 h wird 1 Äquivalent H₂N-Lₙ-R⁵, gelöst in einem organischen Lösungsmittel, zugegeben und 4-24h bei 20°C gerührt. Über beispielsweise Kristallisation oder chromatographische Reinigung wird das Produkt der Formel (A9b) erhalten.

### Variante B:

1 Äquivalent des Amines (A8) wird zusammen mit 1-3 Äquivalenten eines Isocyanates in einem organischen Lösungsmittel beispielesweise Dimethylfromamid, THF, Dimethylacetamid gelöst und 1-24 h bei 40-70°C gerührt.
Nach Entfernen des Lösungsmittels wird das Produkt (A9b) beispielsweise über Kristallisation oder chromatographische Reinigung erhalten.

### Herstellung der Urethane (A9c):

Die in Schema 5A genannten Verbindungen A9c stellen Verbindungen dar, in denen L für eine -O- Gruppe steht. Die nachstehend beschriebenen Vorgehensweisen sind, wie für den Fachmann klar ersichtlich, auch anwendbar, wenn L nicht lediglich O, sondern beispielsweise -O-Alkylen bedeutet.

1 Äquivalent des Amines (A7) wird in organischem Lösungsmittel beispielsweise Dichlormethan, Dimethylformamid, THF gelöst und mit 1-3 Äquivalenten Base beispielsweise Diisopropylethylamin, Triethylamin versetzt. Anschliessend wird 1-3 Äquivalente eines Chlorformiats zugegeben und für 1-24 h bei 20-60°C gerührt. Nach Entfernen des Lösungsmittels wird das Produkt (A9c) beispielsweise über Kristallisation oder chromatographische Reinigung erhalten

Wie in Schema 5A ersichtlich, sind für die Synthese der erfindungsgemäßen Verbindungen der allgemeinen Formel (I) die Verbindungen der Formel (A8) von zentraler Bedeutung. Dementsprechend betrifft die vorliegende Erfindung ferner Zwischenverbindungen der allgemeinen Formel (A8) 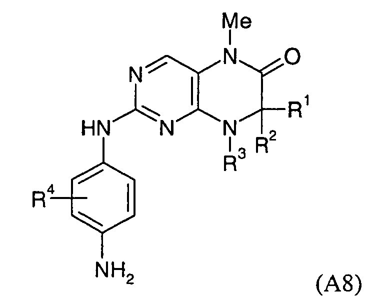
worin die Reste R¹, R², R³ und R⁴ die vorstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

Als Säureadditionssalze kommen hierbei insbesondere diejenigen Salze in Betracht, die vorstehend für die Verbindungen der Formel (I) als pharmakologisch verträgliche Säureadditionssalze aufgeführt werden.

Wie in Schema 5A ersichtlich, sind für die Synthese der erfindungsgemäßen Verbindungen der allgemeinen Formel (I) ferner die Verbindungen der Formel (A7a) als Ausgangsverbindungen von großer Bedeutung. Dementsprechend betrifft die vorliegende Erfindung ferner Zwischenverbindungen der allgemeinen Formel (A7a) 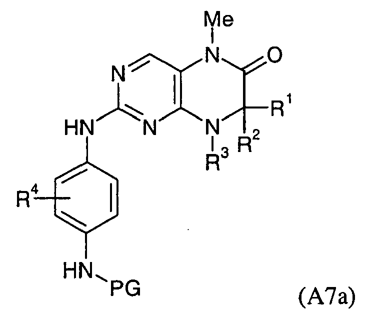
worin die Reste R¹, R², R³ und R⁴ die vorstehend genannten Bedeutungen haben können und worin PG für eine Aminoschutzgruppe steht, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

Bevorzugt sind Verbindungen der allgemeinen Formel (A7a), worin
- PG: ausgewählt ist aus der Gruppe bestehend aus tert-Butyloxycarbonyl-, Acetyl, Trifluormethyl, 9-Fluorenylmethyloxycarbonyl, Allyloxycarbonyl und Benzyloxycarbonyl, bevorzugt tert-Butyloxycarbonyl-, Acetyl und Trifluormethyl,
bedeutet, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

Wie in Schema 5A ersichtlich, sind für die Synthese der erfindungsgemäßen Verbindungen der allgemeinen Formel (I) ferner die Verbindungen der Formel (A7b) als Ausgangsverbindungen von großer Bedeutung. Dementsprechend betrifft die vorliegende Erfindung ferner Zwischenverbindungen der allgemeinen Formel (A7b) 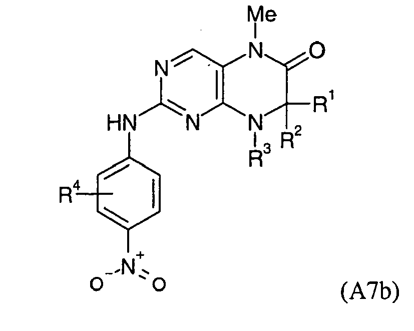
worin die Reste R¹, R², R³ und R⁴ die vorstehend genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

Die Herstellung eines zur Synthese spezifischer Intermediate der allgemeinen Formel (A8), der Zwischenverbindungen Z1-Z12, eingesetzten Eduktes wird im folgenden beschrieben.

Herstellung von 4-Amino-3-methoxy-phenyl-carbaminsäure tert-butylester: 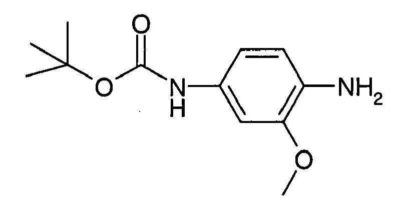 15g 4-Nitro-3-methoxybenzoesäure wurde in 35 g tert-Butanol, 16 mL Disopropylethylamin und 15 mL Toluol gelöst, anschliessend mit 17,5 mL Diphenylphosporylazid versetzt und 7h unter Rückfluss erhitzt. Anschliessend wurde der Ansatz eingeengt und mit 500 mL Essigester versetzt und mit 3x je 200 mL Wasser extrahiert. Die org. Phase wurde getrocknet und die Raektionsmischung über eine Kieselgelchromatographie (Petrolether: Essigester 3:1) getrennt, geeignete Fraktionen wurden vereinigt.
Ausbeute: 16,1 g einer Verbindung B1 (hellgelbe Kristalle)

16g der Verbindung B 1 wurde in 400 mL Ethanol gelöst und mit 6 g 10%iger Pd/C mit Wasserstoff bei 20°C umgesetzt. Die Reaktionslösung wurde eingeengt und der Feststoff mit Diethylether verrieben.
Ausbeute: 10,5 g 4-Amino-3-methoxy-phenyl-carbaminsäure tert-butylester (farblose Kristalle)

Zur Synthese des Beispiels 5 wird zunächst eine Zwischenverbindung Z1
wie im folgenden beschrieben hergestellt. 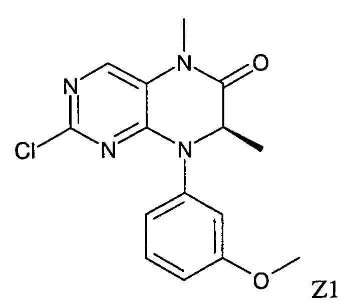 100g Trifluormethansulfonsäureanhydrid wurde in 50 mL Dichlormethan vorgelegt und unter Eiskühlung ein Gemisch aus 39 mL L-Milchsäureethylester und 28 mL Pyridin in 200 mL Dichlormethan in 60 min zugetropft. Anschliessend wurde das Gemsch auf 20 °C erwärmt und nach 1h der ausgefallene Niederschlag abfiltriert. Die Mutterlauge wurde 3x mit je 200 mL Wasser gewaschen und über Natriumsulfat getrocknet und eingeengt.
Ausbeute: 83 g Z1a

43g m-Anisidin und 49 mL Triethylamin wurden in 400 mL Dichlormethan vorgelegt und unter Eiskühlung Z1a gelöst in 200 mL Dichlormethan innerhalb einer Stunde zugetropft. Anschließend wurde die Reaktionsmischung innerhalb von 12 h auf 20°C erwärmt, 3x mit je 200 mL Eiswasser gewaschen, die organische Phase getrocknet und eingeengt. Anschließend wurde das Produkt im Vakuum über eine fraktionierte Destillation gereinigt.
Ausbeute: 49,6 g einer Verbindung Z1b als klares Öl.

48.6 g der Verbindung Z1b in 300 ml Wasser gelöst und 47 g 2,4-Dichlor-5-nitropyrimidin in 300 mL Ether gelöst zugegeben, anschliessend wurde unter Eiskühlung mit 55,6 g wässrige Kaliumhydrogencarbonatlösung zugetropft. Nach 48 h wurden die Phasen getrennt und die org. Phase, über MgSO₄ getrocknet und eingeengt.
Ausbeute: 77.0 g einer Verbindung Z1c (hellrotes Öl)

77 g der Verbindung Z1c wurden in 500 mL Eisessig gelöst und bei 60°C mit 55 g Eisen-Pulver portionsweise versetzt. Es wurde 1 h bei 70°C, dann 45 min bei 100°C gerührt und anschließend heiß über Cellulose abfiltriert. Die Reaktionsmischung wurde mit ca 500 mL Wasser versetzt und der Niederschlag abfiltriert, in Methanol und Ether supendiert und erneut abfiltriert und getrocknet.
Ausbeute: 37.3 g einer Verbindung Z1d (weisse Kristalle)

37.3 g der Verbindung Z1d und 7,7 mL Methyliodid wurden in 250 mL Dimethylacetamid vorgelegt und bei -10°C mit 5.4 g Natriumhydrid als 60%ige Dispersion in Mineralöl versetzt. Es wurde 120 min bei -5°C gerührt, dann auf 500 mL Eiswasser gegeben. Der entstandene Niederschlag wurde abgesaugt und mit Petrolether und Wasser gewaschen. Die noch feuchten Kristalle wurden in Methylenchlorid gelöst, die org. Phase getrocknet und eingeengt. Anschliessend wurde das Gemisch mittels Kieselgelchromatografie getrennt und geeignete Fraktionen vereinigt.
Ausbeute: 25.4 g einer Verbindung Z1e (weisse Kristalle)

1,5 g Z1e und 1,22g 4-Amino-3-methoxy-phenyl-carbaminsäure tert-butylester wurde bei 120°C für 5h zusammengeschmolzen. Nach dem Abkühlen wurde die Reaktionsmischung in Dichlormethan gelöst und 2x mit Kaliumcarbonatlösung und 2x mit Wasser extrahiert. Nach dem Trocknen der org. Phase wurde die Mischung mittels Kieselgelchromatographie (Fliessmittel 99:1, CH2Cl2 : MeOH) getrennt und die geeigneten Produktfraktionen vereinigt.
Ausbeute: 0,92 g hellbraune Kristalle einer Verbindung Z1f

0,92 g Z1f wurden in 100 mL Methylenchlorid gelöst, 15 mL Trifluoressigsäure zugegeben und für 3 h bei 20°C gerührt. Dann wurde die Lösung in eine Mischung aus 10g Eis und 100 mL einer 25%igen Ammoniaklösung gegeben und die org . Phase mit Wasser extrahiert und nach dem Trocknen eingeengt. Der Rückstand wurde in Aceton gelöst und mit etherischer HCl versetzt. Die ausgefallenen Kristalle wurde abfiltriert und getrocknet.
Ausbeute: 0,54g hellbraune Kristalle der Zwischenverbindung Z1

Analog zu der beschriebenen Synthese wurden folgende Zwischenstufen hergestellt: 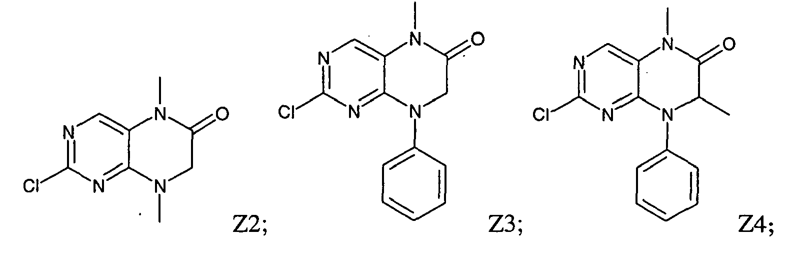 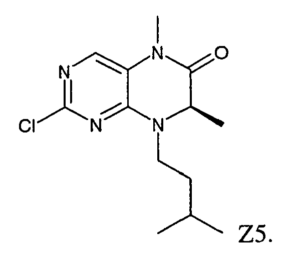 Zur Synthese der Beispiele 1 bis 3 und 13 bis 19 wird zunächst eine Zwischenverbindung Z6 wie im folgenden beschrieben hergestellt. 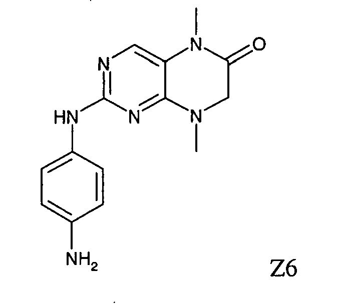 7 g Z2 wurden mit 4,55 g 4-Nitroanilin in 42 mL EtOH, 168 mL Wasser und 1,2 mL 37%iger Salzsäure 24 h bei 90°C gerührt. Die abgekühlte Suspension wurde mit 12 mL 4N NaOH auf pH 10 gestellt und mit 80 mL Dichlormethan versetzt. Der Niederschlag wurde abgesaugt und und die org. Phase eingeengt. Dann wurde der noch feuchte Feststoff in Methanol suspendiert, eingeengt und erneut in Ether suspendiert und der Niederschlag abfiltriert.
Ausbeute 5,09 g einer Zwischenverbindung Z6a als gelber Feststoff.

5,09 g Z6a wurden in 1500 mL Dimethylformamid gelöst und mit 1,5 g Raney-Nickel für 3 h bei 3, 5 bar Wasserstoffdruck und 50°C hydriert. Anschliessend wurde die Reaktionslösung über Kieselgur filtriert und die Lösung eingeengt. Der Rückstand wurde in Ether suspendiert und erneut abfiltriert.
Ausbeute: 4,2 g Z6 (als dunkelgrüner Feststoff)

Zur Synthese des Beispiels 11 wird zunächst eine Zwischenverbindung Z7 wie im folgenden beschrieben hergestellt. 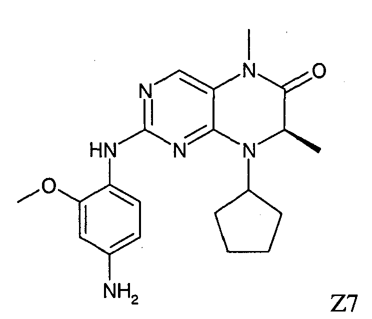 Eine Lösung von128.2 g (0.83 mol) D-Alaninethylester x HCl und 71.5 g (0.85 mol) Cyclopentanon in 1500 mL Dichlormethan wurde mit 70.1 (0.85 mol) Natriumacetat und 265.6 g (1.25 mol) Natriumtriacetoxyborhydrid versetzt. Die Reaktionsmischung wurde 12 h gerührt und dann in 1.5 L einer 10%igen Natriumhydrogencarbonat-Lösung gegossen. Die wässrige Phase wurde mit Dichlormethan extrahiert. Die vereinten organischen Phasen wurden über Na₂SO₄ getrocknet und eingeengt.
Ausbeute: 143.4 g einer Verbindung Z7a (farbloses Öl)

66.0 g der Verbindung Z7a wurden in 500 mL Wasser vorgelegt und mit 85.0 g (0.44 mol) 2,4-Dichlor-5-nitropyrimidin in 500 mL Diethylether versetzt. Bei -5°C wurden 100 mL 10%ige Kaliumhydrogencarbonat-Lösung zugetropft und die Reaktionsmischung 48 h bei Raumtemperatur gerührt. Die wässrige Phase wurde mit Diethylether extrahiert, die vereinten organischen Phasen über Na₂SO₄ getrocknet und eingeengt. Der dunkelrote Feststoff wurde mit Petrolether ausgerührt und abgesaugt.
Ausbeute: 88.0 g einer Verbindung Z7b (gelbe Kristalle)

88.0 g der Verbindung Z7b wurden in 1000 mL Eisessig gelöst und bei 60°C portionsweise mit 85 g Eisen-Pulver versetzt, wobei die Temperatur auf 110°C anstieg. Es wurde 1 h bei 60°C gerührt, anschließend heiß über Cellulose abgesaugt und eingeengt. Der braune Feststoff wurde mit 700 mL Wasser ausgerührt und abgesaugt.
Ausbeute: 53.3 g einer Verbindung Z7c (leicht braune Kristalle)

53.3 g der Verbindung Z7c wurden in 300 mL Dimethylacetamid gelöst und mit 13 mL (0.21 mol) Methyliodid versetzt. Bei -5°C wurden 5.0 g (0.21 mol) Natriumhydrid als 60%ige Dispersion in Mineralöl portionsweise zugegeben. Nach 12 h wurde das Reaktionsgemisch auf 1000 mL Eiswasser gegossen und der entstandene Niederschlag abgesaugt.
Ausbeute: 40.0 g einer Verbindung Z7d (farblose Kristalle)

1,95 g Z7d und 1,66 g 4-Amino-3-methoxy-phenyl-carbaminsäure tert-butylester wurde bei 120°C für 4,5h zusammengeschmolzen. Nach dem Abkühlen wurde die Reaktionsmischung in Dichlormethan gelöst und 1x mit Kaliumcarbonat-Lösung und 2x mit Wasser extrahiert. Nach dem Trocknen der org. Phase wurde die Mischung mittels Kieselgelchromatographie (Fliessmittel 99:1, CH2Cl2 : MeOH) getrennt und die Produktfraktionen vereinigt.
Ausbeute: 1,76 g der Verbindung Z7e (brauner Feststoff)

1,75 g Z7e wurde in 100 mL Methylenchlorid gelöst und die Lösung mit 20 mL Trifluoressigsäure versetzt. Nach 12 h Rühren bei 25°C wurde der Reaktionsansatz unter Kühlung auf halbkonzentrierte Ammoniak-Lösung gegeben und die org. Phase abgetrennt und mit Wasser extrahiert. Nach dem Entfernen des Lösungsmittels wurde in Aceton gelöst und mit etherischer HCl versetzt. Der entstandene Niederschlag wurde abfiltriert und getrocknet.
Ausbeute: 1,32 g der Zwischenverbindung Z7

Zur Synthese des Beispiels 10 wird zunächst eine Zwischenverbindung Z8 wie im folgenden beschrieben hergestellt. 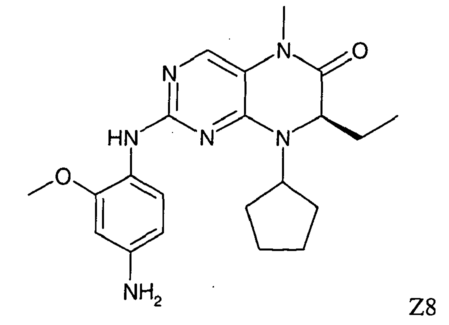 54.0 g (0.52 mol) D-2-Aminobuttersäure wurden in 540 mL Methanol suspendiert und unter Eiskühlung langsam mit 132 g (1.1 mol) Thionylchlorid versetzt. Es wurde 1.5 h unter Rückfluß gekocht und anschließend eingeengt. Das verbliebene Öl wurde mit 540 mL tert-Butylmethylether versetzt und die entstandenen farblosen Kristalle abgesaugt.
Ausbeute: 78.8 g einer Verbindung Z8a (farblose Kristalle)

74.2 g der Verbindung Z8a und 43.5 mL (0.49 mol) Cyclopentanon wurden in 800 mL Dichlormethan gelöst. Nach Zugabe von 40.0 g (0.49 mol) Natriumacetat und 150.0 g (0.71 mol) Natriumtriacetoxyborhydrid bei 0°C wurde 12 h bei Raumtemperatur gerührt und dann 500 mL 20%ige Natriumhydrogencarbonat-Lösung zugegeben. Die wässrige Phase wurde mit Dichlormethan extrahiert. Die vereinten organischen Phasen wurden mit Wasser gewaschen, über MgSO₄ getrocknet und eingeengt.
Ausbeute: 85.8 g einer Verbindung Z8b (leicht gelbes Öl)

40.0 g der Verbindung Z8b und 30.0 g (0.22 mol) Kaliumcarbonat wurden in 600 mL Aceton suspendiert und unter Eiskühlung mit 45.0 g (0.23 mol) 2,4-Dichlor-5-nitropyrimidin in 200 mL Aceton versetzt. Nach 12 h wurden weitere 5.0 g 2,4-Dichlor-5-nitropyrimidin zugegeben und 3 h gerührt. Die Reaktionsmischung wurde eingeengt, in 800 mL Essigsäureethylester und 600 mL Wasser aufgenommen und die wässrige Phase mit Essigsäureethylester extrahiert. Die vereinten organischen Phasen wurden mit Wasser gewaschen, über MgSO₄ getrocknet und eingeengt.
Ausbeute: 75.0 g einer Verbindung Z8c (braunes Öl)

100 g der Verbindung Z8c wurden in 650 mL Eisessig gelöst und bei 70°C mit 20 g Eisen-Pulver portionsweise versetzt. Es wurde 1 h bei 70°C, dann 1.5 h bei 100°C gerührt und anschließend heiß über Kieselgur abfiltriert. Die Reaktionsmischung wurde eingeengt, in Methanol/Dichlormethan aufgenommen, auf Kieselgel aufgezogen und durch Soxhlet-Extraktion mit Essigsäureethylester gereinigt. Das Lösungsmittel wurde entfernt und der Rückstand mit Methanol ausgerührt.
Ausbeute: 30.0 g einer Verbindung Z8d (hellbraune Kristalle)

25.0 g der Verbindung Z8d und 6.5 mL (0.1 mol) Methyliodid wurden in 250 mL Dimethylacetamid vorgelegt und bei -10°C mit 3.8 g (0.95 mol) Natriumhydrid als 60%ige Dispersion in Mineralöl versetzt. Es wurde 20 min bei 0°C, dann 30 min bei Raumtemperatur gerührt und schließlich Eis zugegeben. Die Reaktionsmischung wurde eingeengt und mit 300 mL Wasser versetzt. Der entstandene Niederschlag wurde abgesaugt und mit Petrolether gewaschen.
Ausbeute: 23.0 g einer Verbindung Z8e (farbloser Feststoff)

1,5 g Z8e und 1,22 g 4-Amino-3-methoxy-phenyl-carbaminsäure tert-butylester wurde bei 120°C für 5h zusammengeschmolzen. Nach dem Abkühlen wurde die Reaktionsmischung in Dichlormethan gelöst und 2x mit Kaliumcarbonat-Lösung und 2x mit Wasser extrahiert. Nach dem Trocknen der org. Phase wurde die Mischung mittels Kieselgelchromatographie (Fliessmittel 99:1, CH₂Cl₂ : MeOH) getrenntund die Produktfraktionen vereinigt.
Ausbeute: 0,92 g einer Verbindung Z8f (hellbraune Kristalle)

0,92 g Z8f wurden in 100 mL Methylenchlorid gelöst, 15 mL Trifluoressigsäure zugegeben und für 3 h bei 20°C gerührt. Dann wurde die Lösung in eine Mischung aus 10g Eis und 100 mL einer 25%igen Ammoniak-Lösung gegeben und die org . Phase mit Wasser extrahiert und nach dem Trocknen eingeengt. Der Rückstand wurde in Aceton gelöst und mit etherischer HCl versetzt. Die ausgefallenen Kristalle wurden abfiltriert und getrocknet.
Ausbeute: 0,54g der Zwischenverbindung Z8 (hellbraune Kristalle)

Zur Synthese des Beispiels 8 wird zunächst eine Zwischenverbindung Z9 wie im folgenden beschrieben hergestellt. 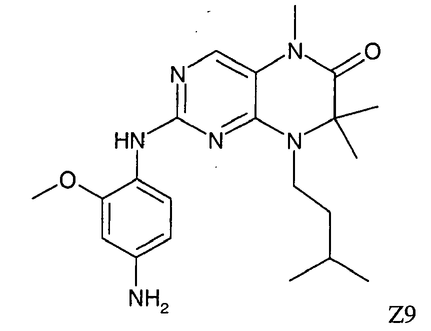 Eine Mischung aus 73.4 mL 2-Bromisobuttersäureethylester, 87.1 mL 3-Methyl-1-butylamin, 82.5 g (0.6 mol) Natriumiodid und 76.0 g (0.6 mol) Kaliumcarbonat in 1000 mL Essigsäureethylester wurde 3 Tage unter Rückfluss gekocht. Vorhandene Salze wurden abfiltriert und das Filtrat eingeengt.
Ausbeute: 97.0 g einer Verbindung Z9a (rotes Öl)

49.0 g 2,4-Dichlor-5-nitropyrimidin und 38.3 g Kaliumcarbonat wurden in 500 mL Aceton suspendiert und bei 0°C mit 93.0 g der Verbindung Z9a in 375 mL Aceton versetzt. Die Reaktionsmischung wurde über Nacht bei Raumtemperatur gerührt, filtriert und eingeengt. Der in Essigsäureethylester gelöste Rückstand wurde mit Wasser gewaschen und die organische Phase über MgSO₄ getrocknet und eingeengt.
Ausbeute: 102.7 g einer Verbindung Z9b (braunes Öl)

22.7 g der Verbindung Z9b wurden in 350 mL Eisessig gelöst und bei 60°C mit 17.4 g Eisen-Pulver portionsweise versetzt. Nach beendeter Zugabe wurde 0.5 h unter Rückfluss gekocht, heiß abfiltriert und eingeengt. Der Rückstand wurde in 200 mL Dichlormethan/Methanol (9:1) aufgenommen und mit Natriumchlorid-Lösung gewaschen. Die organische Phase wurde über Kieselgur abgesaugt, über MgSO₄ getrocknet, eingeengt und mittels Säulenchromatographie (Laufmittel: Essigsäureethylester/Cyclohexan 1:1) getrennt und geeignete Fraktionen vereinigt.
Ausbeute: 1.9 g einer Verbindung Z9c (farblose Kristalle)

1.9 g der Verbindung Z9c wurden in 32 mL Dimethylacetamid gelöst und unter Eiskühlung mit 0.3 g (7 mmol) Natriumhydrid als 60%ige Dispersion in Mineralöl versetzt. Nach 10 min wurden 0.5 mL (7 mmol) Methyliodid zugegeben und 3 h bei Raumtemperatur gerührt. Die Reaktionsmischung wurde eingeengt und mit Wasser versetzt. Der entstandene Niederschlag wurde abgesaugt und mit Petrolether gewaschen.
Ausbeute: 1.6 g einer Verbindung Z9d (farblose Kristalle)

1,5 g Z9d und 1,21 g 4-Amino-3-methoxy-phenyl-carbaminsäure tert-butylester wurde bei 120°C für 4,5h zusammengeschmolzen. Nach dem Abkühlen wurde die Reaktionsmischung in Dichlormethan gelöst und 1x mit Kaliumcarbonat-Lösung und 1x mit Wasser extrahiert. Nach dem Trocknen der org. Phase wurde die Mischung mittels Kieselgelchromatographie (Fliessmittel 98:2, CH₂Cl₂ : MeOH) getrennt und die Produktfraktionen vereinigt.
Ausbeute: 1,12 g einer Verbindung Z9e (hellbrauner Feststoff)

1,12 g Z9e wurde in 100 mL Methylenchlorid gelöst, 18 mL Trifluoressigsäure zugegeben und für 12 h bei 20°C gerührt. Dann wurde die Lösung auf eine halbkonz. Ammoniaklösung gegeben und die org. Phase mit Wasser extrahiert und eingeengt.
Ausbeute: 0,84g der Zwischenverbindung Z9 (beiger Feststoff)

Analog zu den beschriebenen Synthesen wurden folgende Zwischenstufen hergestellt:

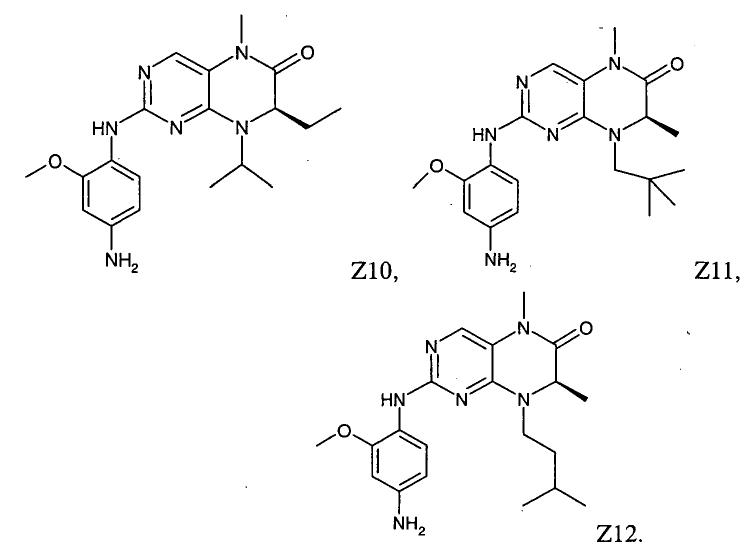

Die neuen Verbindungen der allgemeinen Formel (I) können in Analogie zu nachfolgenden Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken.

### Synthese der Beispiele

### Beispiel 2

0,4 g Z6 wurden in 8 mL Dichlormethan und 2 mL Chloroform suspendiert und mit 0,25 mL 3-Phenylpropionsäurechlorid und mit 0,11 mL Pyridin versetzt. Nach 5 h wurde 10 mL Wasser zugegeben und der Niederschlag mit Wasser und Dichlomethan nachgewaschen. Ausbeute: 0,50 g als grauer Feststoff

### Beispiel 4

0,5 g Z3, 0,547 g 4-Aminoacetanilid wurden in 2 mL Sulfolan für 1h bei 160°C erhitzt. Nach dem Abkühlen wurde Ether und Ethylacetat zugegeben und der enstandene Feststoff abfiltriert. Anschliessend wurde 2x mit Methanol, Aceton und Ethylacetat suspendiert und der Feststoff abfiltriert.
Ausbeute: 0,24 g als weisse Kristalle

### Beispiel 8

0,1 g Z9, 79 mg 4-(4-methyl-piperazin-1-yl)-benzoesäurechlorid, 0,2 mL Triethylamin wurden 2 h in 2 mL Dichlormethan bei 20°C gerührt, dann wurde die org. Phase mit 20 mL 5%iger wässriger Kaliumcarbonat-Lösung extrahiert. Die org. Phase wurde eingeengt und die Mischung durch eine Kieselchromatographie getrennt. Die geeigneten Fraktionen wurden vereinigt, eingeengt und der Rückstand aus Ethylacetat, Diethylether und Petrolether kristallisiert.
Ausbeute: 0,095 g weisse Kristalle

### Beispiel 11

0,1 g Z7, 70 mg 4-(4-methyl-piperazin-1-yl)-benzoesäurechlorid, 0,3 mL Triethylamin wurden 2 h in 2 mL Dichlormethan bei 20°C gerührt, dann wurde die org. Phase mit 20 mL 5%iger wässriger Kaliumcarbonat-Lösung extrahiert. Die org. Phase wurde eingeengt und die Mischung durch eine Kieselchromatographie getrennt. Die geeigneten Fraktionen wurden vereinigt, eingeengt und der Rückstand aus Ethylacetat und Petrolether kristallisiert.
Ausbeute: 0,025 g weisse Kristalle

### Beispiel 15

0,4 g Z6 wurde zusammen mit 0,54 g 4-Ethoxycarbonylphenylisocyanat in 8 mL Dimethylfromamid gelöst und 3 h bei 60°C gerührt. Anschliessend wurde das Lösungsmittel entfernt, der Rückstand in Methylenchlorid suspendiert, abfiltriert, der Rückstand nochmal in Ether suspendiert und erneut abfiltriert.
Ausbeute: 0,37g eines grauen Feststoffes.

### Beispiel 18

0,3 g Z6 wurde in 4 mL Dichlormethan suspendiert und mit 0,2 mL Diisopropylethylamin versetzt. Anschliessend wurde 0,15 mL Phenylchlorformiat, gelöst in 2 mL Dichlormethan, zugetropft und für 4h bei 20°C gerührt. Anschliessend wurde der Feststoff abfiltriert und mit Dichlormethan nachgewaschen.
Ausbeute: 0,26 g als grauer Feststoff

### Beispiel 20

0,32g der Verbindung aus Beispiel 15 wurde in 3 mL Ethanol suspendiert und mit 2 mL 1N NaOH versetzt. Anschliessend wurde für 2,5 h auf Rückfluss erhitzt. Die abgekühlte Suspension wurde mit 5 mL Wasser versetzt und mit 2 mL 4N HCI auf ca pH 1 eingestellt. Die Suspension wurde abfiltriert und mit Wasser und MeOH gewaschen.
Ausbeute: 0,25g als grauer Feststoff

### Beispiel 22

0,1 g Z5 und 0,09 g 4-Amino-3-methoxy-phenyl-carbaminsäure tert-butylester wurden bei 120°C ohne Lösungsmittel für 4h erhitzt. Anschliessend wurde in Dichlormethan aufgenommen und mit Wasser extrahiert. Die organische Phase wurde eingeengt und die Mischung mittels Kieselgelchromatographie getrennt. Die geeigneten Fraktionen wurden vereinigt und erneut eingeengt.
Ausbeute: 0,06 g

Analog zu vorstehend beschriebenen Vorgehensweisen werden u.a. die in Tabelle 1 aufgeführten Verbindungen der Formel (I) erhalten.

Die vorliegende Erfindung betrifft in besonders bevorzugten Ausführungsformen, die Verbindungen der Formel (I), wie sie in Tabelle 1 genannt sind, als solche, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

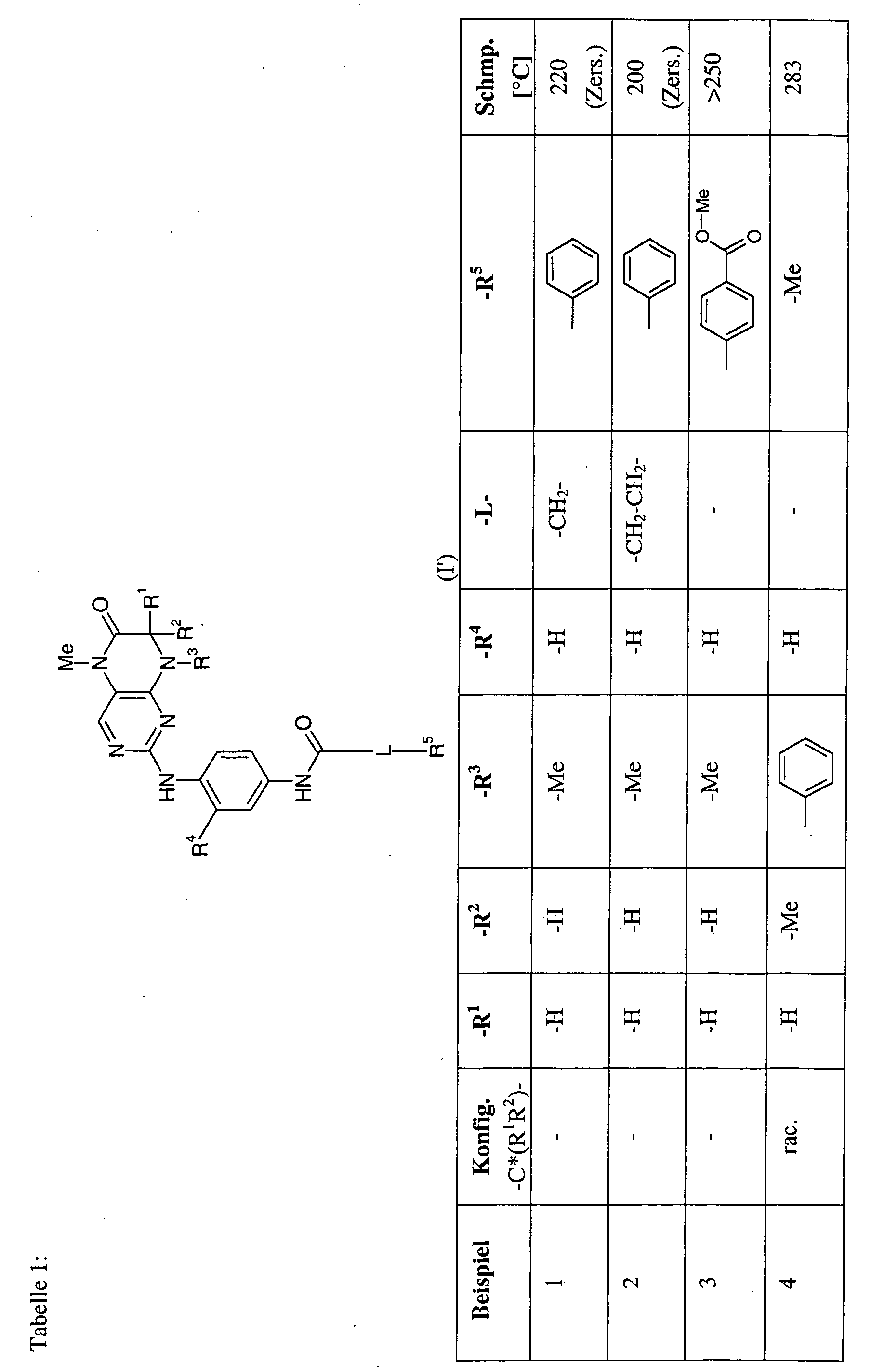

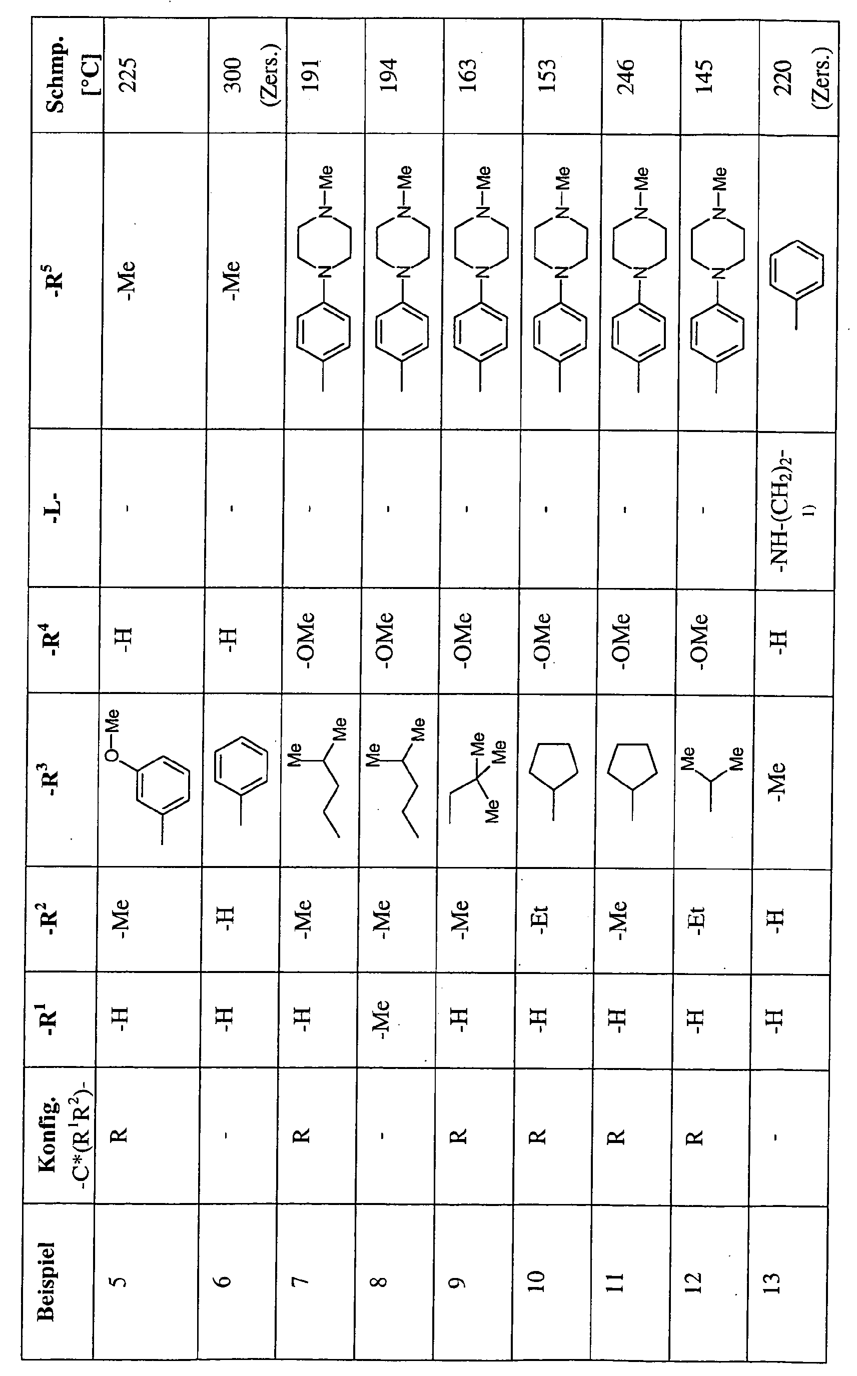

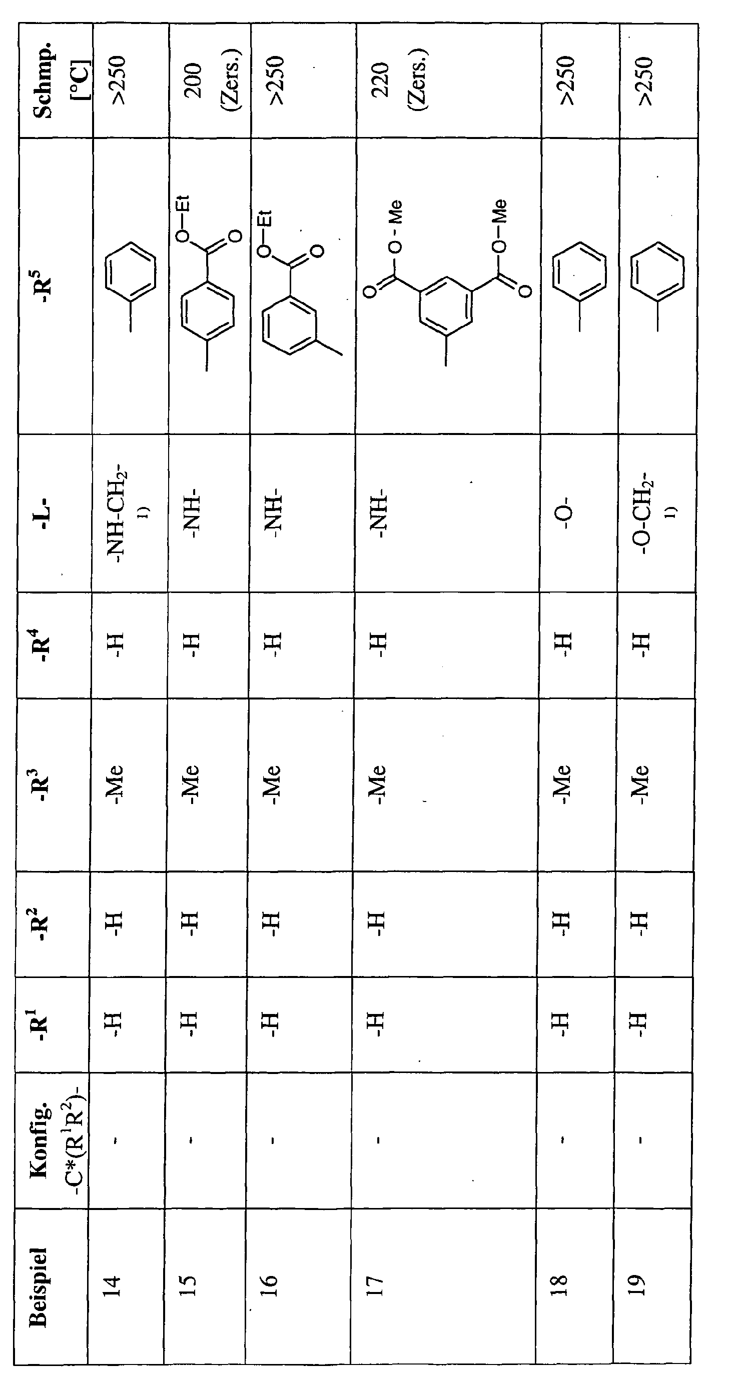

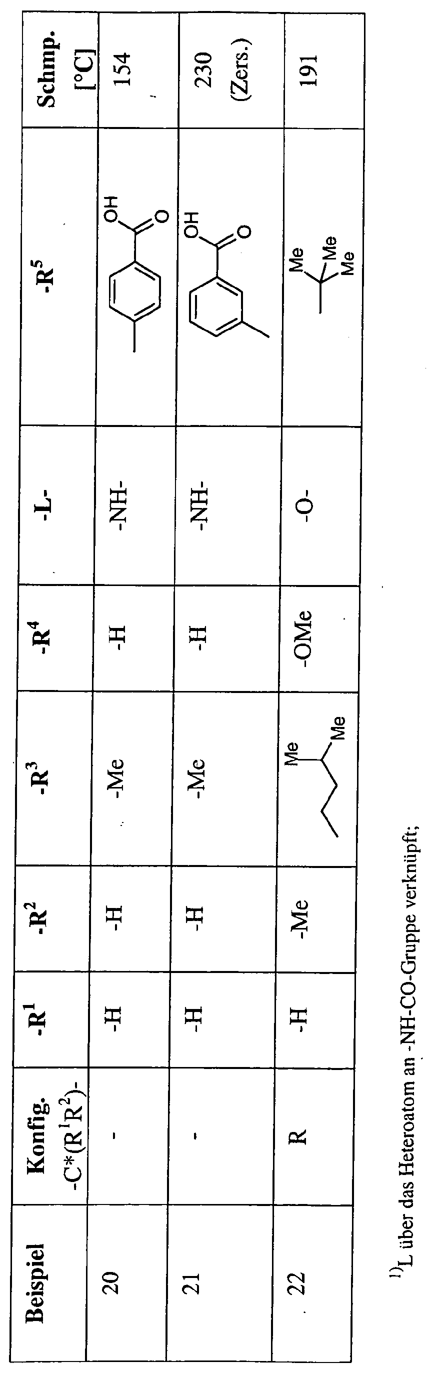

Wie gefunden wurde, zeichnen sich die Verbindungen der allgemeinen Formel (I) durch vielfältige Anwendungsmöglichkeiten auf therapeutischem Gebiet aus. Hervorzuheben sind solche Anwendungsmöglichkeiten, für welche die Inhibition spezifischer Zellzykluskinasen, insbesondere die inhibierende Wirkung auf die Proliferation kultivierter humaner Tumorzellen, aber auch auf die Proliferation anderer Zellen, wie z.B. Endothelzellen, eine Rolle spielen.

Wie durch DNA-Färbung mit darauf folgender FACS Analyse gezeigt werden konnte, ist die, durch die erfindungsgemäßen Verbindungen bewirkte, Proliferationsinhibition vermittelt durch einen Arrest der Zellen vor allem in der G2/M Phase des Zellzyklus. Die Zellen arretieren abhängig von den verwendeten Zellen für eine bestimmte Zeitspanne in dieser Zellzyklus Phase, bevor der programmierte Zelltod eingeleitet wird. Ein Arrest in der G2/M Phase des Zellzyklus wird z.B. durch die Inhibition spezifischer Zellzykluskinasen ausgelöst. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; HautErkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001).
Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Cytostatika, Hormone oder Antikörper verwendet werden.

Die Wirkung der erfindungsgemäßen Verbindungen wurde im PLK1 Inhibitionsassay, im Cytotoxizitätstest an kultivierten humanen Tumorzellen und/oder in einer FACS-Analyse, beispielsweise an HeLa S3-Zellen, bestimmt. Die Verbindungen zeigten in beiden Testmethoden eine gute bis sehr gute Wirksamkeit, d.h. beispielsweise einen EC₅₀-Wert im HeLa S3-Cytotoxizitätstest kleiner 5 µmol/L, in der Regel kleiner 1 µmol/L und einen IC₅₀-Wert im PLK1-Inhibitionsassay kleiner 1 µmol/L.

### PLK1 Kinaseassay

### Enzymherstellung:

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10⁷ Sf21 Zellen (Spodoptera frugiperda) in 200 ml Sf-900 II Serum freien Insektenzellmedium (Life Technologies) werden in eine Spinnerflasche ausgesät. Nach 72 Stunden Inkubation bei 27°C und 70 rpm werden 1x10⁸ Sf21 Zellen in insgesamt 180 ml Medium in eine neue Spinnerflasche ausgesät. Nach weiteren 24 Stunden werden 20 ml rekombinanter Baculovirus Stammsuspension zugesetzt und die Zellen 72 Stunden bei 27°C bei 70 rpin kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4°C, 800 rpm) und 1x mit PBS (8 g NaCl/l, 0,2 g KCl/l, 1,44 g Na₂HPO₄/l, 0,24 g KH₂PO4/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM MgCl₂, 1 mM DTT, 5 µg/ml Leupeptin, 5 µg/ml Aprotinin, 100 µM NaF, 100 µM PMSF, 10 mM β-Glycerolphosphat, 0.1 mM Na₃VO₄, 30 mM 4-Nitrophenylphosphate) zu 1x10⁸ Zellen/ 17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer: 100 mM Tris/HCI pH=8,0, 120 mM NaCl, 20 mM reduziertes Glutathion (Sigma G-4251), 10 mM MgCl₂, 1 mM DTT) von den Beads eluiert. Die Proteinkonzentration wird mittels Bradford Assay bestimmt.

### Assaydurchführung:

In einem Napf einer 96-Loch Rundbodenplatte (Fa. Greiner bio-one, PS-Microtiterplatte Nr.650101) werden folgende Komponenten zusammengefügt:
- 10 µl zu testende Verbindung in variabler Konzentration (z.B. beginnend bei 300 µM, und Verdünnung in 1:3) in 6% DMSO, 0,5 mg/ml Casein (Sigma C-5890), 60 mM β-Glycerophosphat, 25 mM MOPS pH=7,0, 5 mM EGTA, 15 mM MgCl₂, 1 mM DTT
- 20 µl Substratlösung (25 mM MOPS pH=7,0, 15 mM MgCl₂, 1 mM DTT, 2,5 mM EGTA, 30 mM β-Glycerophosphat, 0,25 mg/ml Casein)
- 20 µl Enzymverdünnung (1:100 Verdünnung des Enzymstocks in 25 mM MOPS pH=7,0, 15 mM MgCl₂, 1 mM DTT)
- 10 µl ATP Lösung (45 µM ATP mit 1,11x10⁶ Bq/ml gamma-P33-ATP).
Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1%iger TCA gewaschen und bei 60°C getrocknet. Nach Zugabe von 35µl Szintillationslösung (Ready-Safe; Beckmann) pro Napf wird die Platte mit Sealing-tape zugeklebt und die präzipitierte Menge P33 mit dem Wallac Betacounter gemessen.
Die Messdaten werden mit der Standard Graphpad Software (Levenburg-Marquard Algorhythmus) ausgewertet.

### Messung der Cytotoxizität an kultivierten humanen Tumorzellen

Zur Messung der Cytotoxizität an kultivierten humanen Tumorzellen wurden Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend wurden die HeLa S3 Zellen in 96-well Platten (Costar) mit einer Dichte von 1000 Zellen pro well eingebracht und über Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue Reagenz). Die Wirksubstanzen wurden in verschiedenen Konzentrationen (gelöst in DMSO; DMSO-Endkonzentration: 0.1 %) zu den Zellen zugegeben (jeweils als Dreifachbestimmung). Nach 72 Stunden Inkubation wurden zu jeden well 20 µl AlamarBlue Reagenz(AccuMed International) zugesetzt, und die Zellen für weitere 7 Stunden inkubiert. Zur Kontrolle wurde zu 3 wells je 20 µl reduziertes AlamarBlue Reagenz gegeben (AlamarBlue Reagenz, das für 30 min autoklaviert wurde). Nach 7 h Inkubation wurde der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wurde in Prozent der Kontrolle (HeLa S3 Zellen ohne Inhibitor) berechnet und die Wirkstoffkonzentration, die die Zellaktivität zu 50% hemmt (IC₅₀) abgeleitet. Die Werte wurden hierbei aus dem Mittelwert von drei Einzelbestimmungen - unter Korrektur des Leerwertes (Mediumkontrolle)- berechnet.

### FACS- Analyse

Propidium Iodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N) , während Zellen in der G2 oder Mitose einen 4N DNA Gehalt haben.
Für eine PI-Färbung wurden beispielsweise 0.4 Mio HeLa S3 Zellen auf eine 75 cm² Zellkulturflasche ausgesät, nach 24 h wurde entweder 0.1 % DMSO als Kontrolle zugesetzt, bzw. die Substanz in verschiedenen Konzentrationen (in 0.1% DMSO). Die Zellen wurden für 24 h mit der Substanz bzw. mit DMSO inkubiert, bevor die Zellen 2 x mit PBS gewaschen und dann mit Trypsin /EDTA abgelöst wurden. Die Zellen wurden zentrifugiert (1000 Upm, 5 min, 4°C), und das Zellpellet 2 x mit PBS gewaschen, bevor die Zellen in 0.1 ml PBS resuspendiert wurden. Anschließend wurden die Zellen für 16 Stunden bei 4°C oder alternativ für 2 Stunden bei -20°C mit 80% Ethanol fixiert. Die fixierten Zellen (10⁶ Zellen) wurden zentrifugiert (1000 Upm, 5min, 4°C) , mit PBS gewaschen und anschließend nochmals zentrifugiert . Das Zellpellet wurde in 2 ml Triton X-100 in 0.25 % PBS resuspendiert, und 5 min auf Eis inkubiert, bevor 5 ml PBS zugeben wurden und erneut zentrifugiert wurde. Das Zellpellet wurde in 350 µl PI Färbelösung (0.1 mg/ml RNase A, 10 µg/ml Prodium Iodid in 1 x PBS) resuspendiert. Die Zellen wurden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einen Argonlaser (500 mW, Emission 488 nm), und dem DNA Cell Quest Programm (BD). Die logarithmische PI Fluoreszenz wurde mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend, wurden erfindungsgemäße Verbindungen auf weiteren Tumorzellen getestet. Beispielsweise sind diese Verbindungen auf Karzinomen verschiedenster Gewebe (z. Bspl. Brust (MCF7); Colon (HCT116), Kopf-Hals (FaDu), Lunge (NCI-H460), Pankreas (BxPC-3), Prostata (DU145)), Sarkome (z. Bspl. SK-UT-1B), Leukämien und Lymphome (z. Bspl. HL-60; Jurkat, THP-1) und anderen Tumoren (z. Bspl. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.
Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Ein weiterer Aspekt der vorliegenden Erfindung betrifft pharmazeutische Formulierungen, vorzugsweise pharmazeutische Formulierung der vorstehend genannten Beschaffenheit, gekennzeichnet durch einen Gehalt an einer oder mehrerer Verbindungen der allgemeinen Formel (I).

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feuchtgranuliert und getrocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (I) 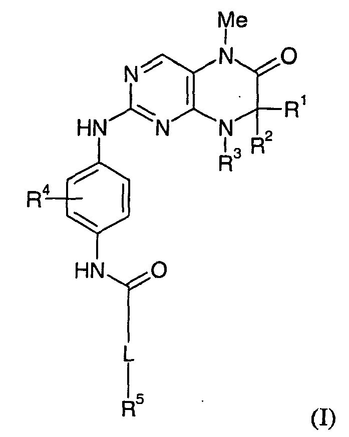
worin
L eine Einfachbindung, oder eine verbrückende zweibindige Gruppe ausgewählt aus der Gruppe bestehend aus C₁-C₆-Alkylen, C₂-C₆-Alkenylen, C₂-C₆-Alkinylen, C₃₋C₇-Cycloalkylen, C₁-C₄-Alkylen-C₆-C₁₀-Arylen-C₁-C₄-Alkylen, C₁-C₄-Alkylen-C₆₋C₁₀-Arylen, -O-, -O-C₁-C₆-Alkylen, -O-C₃-C₆-Alkenylen, -O-C₃-C₆-Alkinylen, -O-C₃-C₇-Cycloalkylen, -O-C₁-C₄-Alkylen-C₆-C₁₀-Arylen-C₁-C₄-Alkylen, -O-C₁-C₄₋Alkylen-C₆-C₁₀-Arylen, -NR⁷- und -NR⁷-C₁-C₆-Alkylen, -NR⁷-C₃-C₆-Alkenylen, - NR⁷-C₃-C₆-Alkinylen, -NR⁷-C₃-C₇-Cycloalkylen, - NR⁷-C₁-C₄-Alkylen-C₆-C₁₀₋Arylen-C₁-C₄-Alkylen, -NR⁷-C₁-C₄-Alkylen-C₆-C₁₀-Arylen, welche gegebenenfalls substituiert sein kann durch einen oder mehrere Reste R⁹;
R¹ und R² , gleich oder verschieden, Wasserstoff, oder ein Rest ausgewählt aus der Gruppe bestehend aus C₁-C₆-Alkyl, C₂-C₆-Alkenyl und C₂-C₆-Alkinyl, der gegebenenfalls ein- oder mehrfach substituiert sein kann durch einen oder mehrere Reste R⁹, oder
R¹ und R² gemeinsam C₂-C₆-Alkylen, in welchem gegebenenfalls ein oder zwei Methylengruppen ersetzt sein können durch eine der Gruppen -O- oder -NR⁷-, und welches gegebenenfalls ein- oder mehrfach substituiert sein kann durch einen oder mehrere Reste R⁹;
R³ Wasserstoff oder ein Rest ausgewählt aus C₁-C₈-Alkyl, C₂-C₈-Alkenyl, C₂-C₈₋Alkinyl, C₃-C₈-Cycloalkyl und C₆-C₁₄-Aryl, der gegebenenfalls ein- oder mehrfach substituiert sein kann durch einen oder mehrere Reste R⁹; oder
R³ und R² oder R³ und R¹ gemeinsam C₂-C₆-Alkylen, das gegebenenfalls ein- oder mehrfach substituiert sein kann durch einen oder mehrere Reste R⁹;
R⁴ Wasserstoff, Halogen, CN, OH, -NR⁷R⁸ oder ein Rest ausgewählt aus der Gruppe bestehend aus C₁-C₆-Alkyl, C₁-C₆-Alkyloxy, C₂-C₆-Alkenyl, C₂-C₆-Alkenyloxy, C₂-C₆-Alkinyl und C₂-C₆-Alkinyloxy, der gegebenenfalls ein- oder mehrfach substituiert sein kann durch einen oder mehrere Reste R¹⁰;
R⁵ Phenyl, dass gegebenenfalls ein oder mehrfach substituiert sein durch einen oder mehrere Reste R¹¹, oder
R⁵ Phenyl, dass einfach substituiert ist durch einen Rest R⁶, oder
R⁵ C₁-C₄-Alkyl, das gegebenenfalls ein oder mehrfach substituiert sein kann durch einen oder mehrere Reste R⁹, oder
R⁶ -NR⁷R⁸ oder eine 5-10 gliedrige Heterocycloalkylgruppe, die ein-, zwei oder drei Heteroatome ausgewählt aus der Gruppe Stickstoff, Sauerstoff und Schwefel, bevorzugt Stickstoff oder Sauerstoff, enthalten kann und die gegebenenfalls ein- oder mehrfach substituiert sein kann durch einen oder mehrere der Reste R¹²;
R⁷ und R⁸, gleich oder verschieden, Wasserstoff oder C₁-C₆-Alkyl,
R⁹ Halogen, C₁-C₄-Alkyl, C₁-C₄-Alkyloxy, CN, OH oder CF₃;
R¹⁰ Halogen, OH, CN, =O, C₁-C₆-Alkyloxy, COOR⁷, NR⁷R⁸, CONR⁷R⁸, SO₂R⁷, CHF₂ oder CF₃;
R¹¹ Halogen, OH, CN, C₁-C₄-Alkyl, C₁-C₄-Alkyloxy, COOR⁷, CONR⁷R⁸, SO₂R⁷, CHF₂, CF₃, C₆-C₁₀-Aryl oder C₁-C₆-Alkylen-C₆-C₁₀-Aryl;
R¹² Halogen, CF₃, C₁-C₆-Alkyl, C₁-C₆-Alkylen-C₆-C₁₀-Aryl, C₃-C₈-Cycloalkyl oder C₁-C₆-Alkylen-C₃-C₈-Cycloalkyl;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

2. Verbindungen der allgemeinen Formel (I) nach Anspruch 1,
worin
L eine Einfachbindung, oder eine verbrückende zweibindige Gruppe ausgewählt aus der Gruppe bestehend aus C₁-C₆-Alkylen, C₂-C₆-Alkenylen, C₂-C₆-Alkinylen, C₃₋C₇-Cycloalkylen, C₁-C₄-Alkylen-C₆-C₁₀-Arylen-C₁-C₄-Alkylen, -O-, -O-C₁-C₄-Alkylen, -NR⁷- und -NR⁷-C₁-C₄-Alkylen, welche gegebenenfalls substituiert sein kann durch einen oder mehrere Reste R⁹;
R¹ und R², gleich oder verschieden, Wasserstoff, oder ein Rest ausgewählt aus der Gruppe bestehend aus C₁-C₆-Alkyl, C₂-C₆-Alkenyl und C₂-C₆-Alkinyl, der gegebenenfalls ein- oder mehrfach substituiert sein kann durch einen oder mehrere Reste R⁹, oder
R¹ und R² gemeinsam C₂-C₆-Alkylen, in welchem gegebenenfalls ein oder zwei Methylengruppen ersetzt sein können durch eine der Gruppen -O- oder -NR⁷-, und welches gegebenenfalls ein- oder mehrfach substituiert sein kann durch einen oder mehrere Reste R⁹;
R³ Wasserstoff oder ein Rest ausgewählt aus C₁-C₈-Alkyl, C₂-C₈-Alkenyl, C₂-C₈₋Alkinyl, C₃-C₈-Cycloalkyl und C₆-C₁₄-Aryl, der gegebenenfalls ein- oder mehrfach substituiert sein kann durch einen oder mehrere Reste R⁹; oder
R³ und R² oder R³ und R¹ gemeinsam C₂-C₆-Alkylen, das gegebenenfalls ein- oder mehrfach substituiert sein kann durch einen oder mehrere Reste R⁹;
R⁴ Wasserstoff, Halogen, CN, OH, -NR⁷R⁸ oder ein Rest ausgewählt aus der Gruppe bestehend aus C₁-C₆-Alkyl, C₁-C₆-Alkyloxy, C₂-C₆-Alkenyl, C₂-C₆-Alkenyloxy, C₂-C₆-Alkinyl und C₂-C₆-Alkinyloxy, der gegebenenfalls ein- oder mehrfach substituiert sein kann durch einen oder mehrere Reste R¹⁰;
R⁵ Phenyl, dass gegebenenfalls ein oder mehrfach substituiert sein durch einen oder mehrere Reste R¹¹, oder
R⁵ Phenyl, dass einfach substituiert ist durch einen Rest R⁶, oder
R⁵ C₁-C₄-Alkyl, das gegebenenfalls ein oder mehrfach substituiert sein kann durch einen oder mehrere Reste R⁹, oder
R⁶ -NR⁷R⁸ oder eine 5-10 gliedrige Heterocycloalkylgruppe, die ein-, zwei oder drei Heteroatome ausgewählt aus der Gruppe Stickstoff, Sauerstoff und Schwefel, bevorzugt Stickstoff oder Sauerstoff, enthalten kann und die gegebenenfalls ein- oder mehrfach substituiert sein kann durch einen oder mehrere der Reste R¹²;
R⁷ und R⁸, gleich oder verschieden, Wasserstoff oder C₁-C₆-Alkyl,
R⁹ Halogen, C₁-C₄-Alkyl, C₁-C₄-Alkyloxy, CN, OH oder CF₃;
R¹⁰ Halogen, OH, CN, =O, C₁-C₆-Alkyloxy, COOR⁷, CONR⁷R⁸, SO₂R⁷, CHF₂ oder CF₃;
R¹¹ Halogen, OH, CN, C₁-C₄-Alkyl, C₁-C₄-Alkyloxy, COOR⁷, CONR⁷R⁸, SO₂R⁷, CHF₂, CF₃, C₆-C₁₀-Aryl oder C₁-C₆-Alkylen-C₆-C₁₀-Aryl;
R¹² Halogen, CF₃, C₁-C₆-Alkyl, C₁-C₆-Alkylen-C₆-C₁₀-Aryl, C₃-C₈-Cycloalkyl oder C₁-C₆-Alkylen-C₃-C₈-Cycloalkyl;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

3. Verbindungen der allgemeinen Formel (I) nach Anspruch 1 oder 2,
worin
Bevorzugt sind ferner Verbindungen der allgemeinen Formel (I), worin
L eine Einfachbindung, -O-; -O-C₁-C₃-Alkylen, -NR⁷-, -NR⁷-C₁-C₃-Alkylen oder C₁-C₄-Alkylen, welches gegebenenfalls substituiert sein kann durch einen oder mehrere Reste R⁹;
R¹ und R², gleich oder verschieden, Wasserstoff, oder ein Rest ausgewählt aus der Gruppe bestehend aus C₁-C₄-Alkyl, C₂-C₄-Alkenyl und C₂-C₄-Alkinyl, der gegebenenfalls ein- oder mehrfach substituiert sein kann durch einen oder mehrere Reste R⁹, oder
R¹ und R² gemeinsam C₂-C₄-Alkylen, das gegebenenfalls ein- oder mehrfach substituiert sein kann durch einen oder mehrere Reste R⁹;
R³ Wasserstoff oder ein Rest ausgewählt aus C₁-C₆-Alkyl, C₃-C₇-Cycloalkyl und C₆₋C₁₀-Aryl, der gegebenenfalls ein- oder mehrfach substituiert sein kann durch einen oder mehrere Reste R⁹; oder
R³ und R² oder R³ und R¹ gemeinsam C₂-C₄-Alkylen, das gegebenenfalls ein- oder mehrfach substituiert sein kann durch einen oder mehrere Reste R⁹;
R⁴ Wasserstoff, Fluor, Chlor, Brom, -NR⁷R⁸ oder ein Rest ausgewählt aus der Gruppe bestehend aus C₁-C₄-Alkyl, C₁-C₄-Alkyloxy, C₂-C₄-Alkenyloxy und C₂-C₄₋Alkinyloxy, der gegebenenfalls ein- oder mehrfach substituiert sein kann durch einen oder mehrere Reste R¹⁰;
R⁵ Phenyl, dass gegebenenfalls ein- oder zweifach substituiert sein durch einen oder mehrere Reste R¹¹, oder
R⁵ Phenyl, dass einfach substituiert ist durch einen Rest R⁶, oder
R⁵ ein Rest ausgewählt aus der Gruppe bestehend aus Methyl, Ethyl, Propyl und Butyl, der gegebenenfalls ein oder zweifach substituiert sein kann durch einen oder mehrere Reste R⁹;
R⁶ ein Heterocycloalkyl ausgewählt aus der Gruppe bestehend aus Piperidinyl, Piperazinyl, Pyrrolinyl, Pyrrolidinyl, Pyrazolinyl, Pyrazolidinyl, Imidazolinyl, Imidazolidinyl und Morpholinyl, das gegebenenfalls ein- oder mehrfach substituiert sein kann durch einen oder mehrere der Reste R¹²;
R⁷ und R⁸, gleich oder verschieden, Wasserstoff oder C₁-C₄-Alkyl,
R⁹ Halogen, C₁-C₄-Alkyl, C₁-C₄-Alkyloxy, CN, OH oder CF₃;
R¹⁰ Halogen, OH, =O, C₁-C₄-Alkyloxy oder CF₃;
R¹¹ Halogen, OH, CN, C₁-C₄-Alkyl, C₁-C₄-Alkyloxy, COOR⁷, CF₃, oder C₁-C₄₋Alkylen-Phenyl;
R¹² C₁-C₄-Alkyl, C₁-C₄-Alkylen-Phenyl, C₃-C₆-Cycloalkyl oder C₁-C₄-Alkylen-C₃-C₆₋Cycloalkyl;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

4. Verbindungen der allgemeinen Formel (I) nach Anspruch 1, 2 oder 3,
worin
L eine Einfachbindung, C₁-C₄-Alkylen, -O-, -O-C₁-C₃-Alkylen, -NH- oder -NH-C₁-C₃-Alkylen;
R¹ und R², gleich oder verschieden, Wasserstoff, oder ein Rest ausgewählt aus der Gruppe bestehend aus C₁-C₄-Alkyl, C₂-C₄-Alkenyl und C₂-C₄-Alkinyl, der gegebenenfalls ein- oder zweifach substituiert sein kann durch einen Rest ausgewählt aus der Gruppe bestehend aus Fluor, Chlor, OH und CF₃; oder
R³ Wasserstoff oder ein Rest ausgewählt aus C₁-C₆-Alkyl, C₃-C₇-Cycloalkyl und C₆₋C₁₀-Aryl, der gegebenenfalls ein- oder zweifach substituiert sein kann durch einen Rest ausgewählt aus der Gruppe bestehend aus Fluor, Chlor, Methyl, Ethyl, OH, Methyloxy, Ethyloxy und CF₃;
R⁴ Wasserstoff, Fluor, Chlor, Brom, -NR⁷R⁸ oder ein Rest ausgewählt aus der Gruppe bestehend aus C₁-C₄-Alkyl, C₁-C₄-Alkyloxy, C₂-C₄-Alkenyloxy und C₂-C₄₋Alkinyloxy, der gegebenenfalls ein- oder zweifach substituiert sein kann durch einen Rest ausgewählt aus der Gruppe bestehend aus Fluor, Chlor, OH, Methoxy, Ethoxy und CF₃;
R⁵ Phenyl, das gegebenenfalls ein- oder zweifach substituiert sein kann durch einen Rest ausgewählt aus der Gruppe bestehend aus Methyl, Ethyl, OH, Fluor, Chlor, CF₃, COOH, COOMethyl oder COOEthyl, oder
R⁵ Phenyl, das einfach substituiert ist durch ein Heterocycloalkyl ausgewählt aus der Gruppe bestehend aus Piperidinyl, Piperazinyl, Pyrrolinyl, Pyrrolidinyl, Pyrazolinyl, Pyrazolidinyl, Imidazolinyl, Imidazolidinyl und Morpholinyl, der ein- oder zweifach substituiert sein kann durch Methyl, Ethyl, Benzyl, Phenethyl, Cyclopropyl oder Cyclopropylmethyl, oder
R⁵ ein Rest ausgewählt aus der Gruppe bestehend aus Methyl, Ethyl, Propyl und Butyl, der gegebenenfalls ein oder zweifach substituiert sein kann durch einen oder mehrere Reste ausgewählt aus der Gruppe Fluor, Chlor, OH und CF₃;
R⁷ und R⁸ , gleich oder verschieden, Wasserstoff, Methyl oder Ethyl, bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

5. Verbindungen der allgemeinen Formel (I) nach einem der Ansprüche 1 bis 4,
worin
L eine Einfachbindung, -CH₂-, -CH₂-CH₂-, -O-, -O-CH₂-, -O-CH₂-CH₂-, -NH-, -NH-CH₂- oder -NH-CH₂-CH₂- ;
R¹ und R², gleich oder verschieden Wasserstoff oder ein Rest ausgewählt aus der Gruppe bestehend aus Methyl, Ethyl, Propyl, Butyl, Allyl und Propargyl, der gegebenenfalls ein- oder zweifach substituiert sein kann durch einen Rest ausgewählt aus der Gruppe bestehend aus Fluor, Chlor und CF₃;
R³ Wasserstoff oder ein Rest ausgewählt aus der Gruppe bestehend aus Methyl, Ethyl, Propyl, Butyl, Pentyl, Cyclopropyl, Cyclopentyl, Cyclohexyl und Phenyl, der gegebenenfalls ein- oder zweifach substituiert sein kann durch einen Rest ausgewählt aus der Gruppe bestehend aus Fluor, Chlor, Methyl, Ethyl, Methyloxy, Ethyloxy und CF₃;
R⁴ Wasserstoff, Methyl, Ethyl, Propyl, Methyloxy, Ethyloxy oder Propyloxy;
R⁵ Phenyl, das gegebenenfalls ein- oder zweifach substituiert sein kann durch einen Rest ausgewählt aus der Gruppe bestehend aus Methyl, OH, Fluor, CF₃, COOH, COOMethyl oder COOEthyl, oder
R⁵ Phenyl, das einfach substituiert ist durch ein Heterocycloalkyl ausgewählt aus der Gruppe bestehend aus Piperidinyl, Piperazinyl, Pyrrolidinyl und Morpholinyl, der ein- oder zweifach substituiert sein kann durch Methyl, Ethyl, Benzyl, Phenethyl, Cyclopropyl oder Cyclopropylmethyl;
R⁵ ein Rest ausgewählt aus der Gruppe bestehend aus Methyl, Ethyl, Propyl und Butyl, der gegebenenfalls ein oder zweifach substituiert sein kann durch einen oder mehrere Reste ausgewählt aus der Gruppe Fluor, Chlor und CF₃;
bedeuten, gegebenenfalls in Form ihrer Tautomeren, ihrer Racemate, ihrer Enantiomere, ihrer Diastereomere und ihrer Gemische, sowie gegebenenfalls in Form ihrer pharmakologisch unbedenklichen Säureadditionssalze, Solvate und oder Hydrate.

6. Pharmazeutische Formulierung enthaltend wenigstens eine Verbindung der Formel (I) nach einem der Ansprüche 1 bis 5.

7. Verbindung der Formel (I) nach einem der Ansprüche 1 bis 5 als Arzneimittel.

8. Verwendung einer Verbindung der Formel (I) nach einem der Ansprüche 1 bis 5 zur Herstellung eines Arzneimittels zur Behandlung von Erkrankungen, die durch exzessive oder anomale Zellproliferation charakterisiert sind.

9. Zwischenverbindungen der allgemeinen Formel (A8) 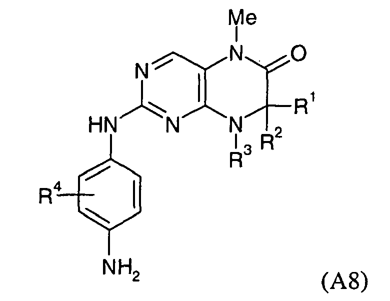
worin die Reste R¹, R², R³ und R⁴ die in den Ansprüchen 1 bis 5 genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

10. Zwischenverbindungen der allgemeinen Formel (A7a) 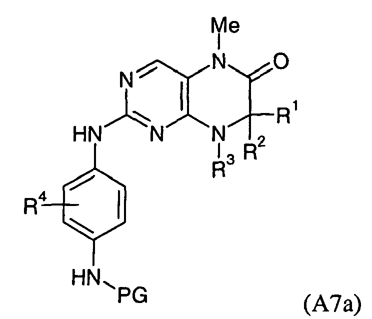
worin die Reste R¹, R², R³ und R⁴ die in den Ansprüchen 1 bis 5 genannten Bedeutungen haben können und worin PG für eine Aminoschutzgruppe steht, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

11. Zwischenverbindungen der allgemeinen Formel (A7b) 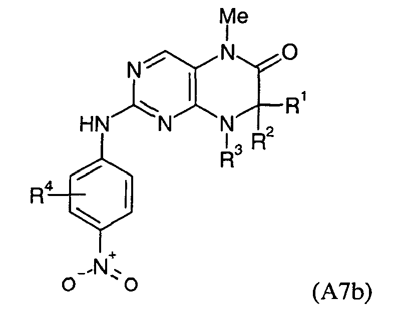
worin die Reste R¹, R², R³ und R⁴ die in den Ansprüchen 1 bis 5 genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.

12. Zwischenverbindungen der Formel (A5) 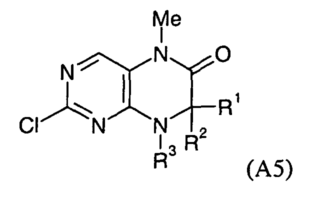
worin die Reste R¹, R² und R³ die in den Ansprüchen 1 bis 5 genannten Bedeutungen haben können, gegebenenfalls in Form ihrer Tautomeren, ihrer Racemate, ihrer Enantiomere, ihrer Diastereomere und ihrer Gemische, sowie gegebenenfalls in Form ihrer Säureadditionssalze, Solvate und oder Hydrate.
